# EUROPÄISCHE PATENTSCHRIFT

(11) **EP 2 949 353 B1**
(45) Veröffentlichungstag und Bekanntmachung des Hinweises auf die Patenterteilung: **11.10.2017**
(21) Anmeldenummer: 15166614.6
(22) Anmeldetag: 21.10.2013
(51) Int. Cl.: A61M 5/00, B65B 35/38, B65B 43/59, B65B 3/00, B65B 3/28, B65B 1/32, G01G 17/06, B01L 9/06, B65B 7/28, B65D 51/24, F26B 5/06, B65B 43/46, B65B 1/46, G01N 35/04

(54) **VERFAHREN UND VORRICHTUNG ZUR BEHANDLUNG ODER VERARBEITUNG VON BEHÄLTERN FÜR SUBSTANZEN FÜR MEDIZINISCHE, PHARMAZEUTISCHE ODER KOSMETISCHE ANWENDUNGEN**
METHOD AND APPARATUS FOR TREATING OR PROCESSING CONTAINERS FOR SUBSTANCES FOR MEDICAL, PHARMACEUTICAL OR COSMETIC USES
PROCÉDÉ ET DISPOSITIF DE TRAITEMENT OU DE MANIPULATION DE RÉCIPIENTS POUR DES SUBSTANCES DESTINÉES À DES APPLICATIONS MÉDICALES, PHARMACEUTIQUES OU COSMÉTIQUES

(30) Priorität: 12.11.2012 DE 102012110866
(43) Veröffentlichungstag der Anmeldung: 02.12.2015
(62) Teilanmeldung aus: 13780316.9
(73) Patentinhaber: Schott AG, 55122 Mainz (DE)
(72) Erfinder: Deutschle, Gregor Fritz, 65185 Wiesbaden (DE); Wassenberg, Jörn, 55130 Mainz (DE); Wansel, Alexander, 65193 Wiesbaden (DE); Wissner, Kai, 69493 Hirschberg (DE)
(74) Vertreter: 2K Patentanwälte Blasberg Kewitz & Reichel

(56) Entgegenhaltungen:
- EP-A1- 1 138 390
- WO-A2-2007/061987
- DE-A1-102004 035 061
- DE-A1-102005 014 116
- US-A1- 2009 158 612

## Beschreibung

Die vorliegende Erfindung betrifft allgemein die gleichzeitige Halterung einer Mehrzahl von Behältern zur Aufbewahrung von Substanzen für medizinische, pharmazeutische oder kosmetische Anwendungen, insbesondere von Fläschchen (Vials), Ampullen oder Karpulen, und betrifft insbesondere ein Verfahren und eine Vorrichtung zur Behandlung oder Verarbeitung bzw. Prozessierung von Fläschchen an oder in einer Bearbeitungsstation, insbesondere während diese von einer Haltestruktur gehalten oder zumindest geführt sind, sowie eine Verstelleinrichtung zu diesem Zweck.

### Hintergrund der Erfindung

Als Behälter zur Aufbewahrung von und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpulen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiterverarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die die Haltestruktur aus dem Transport- und Verpackungsbehälter, die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten werden. Die einzelnen Medikamentenbehälter werden zunächst lose in Aufnahmen, die in der Haltestruktur ausgebildet sind, angeordnet. Anschließend wird die Haltestruktur in den Transport- und Verpackungsbehälter eingesetzt und dieser mit einem gasundurchlässigen Kunststoffschlauch umgeben. Beim anschließenden Evakuieren der so ausgebildeten Verpackungseinheit wird der Kunststoffschlauch aufgrund des in dem Schlauch vorherrschenden Unterdrucks in die Zwischenräume zwischen den Medikamentenbehältern hineingedrückt was so einerseits zu einer Stabilisierung der Position der Medikamentenbehälter in der Haltestruktur führt und andererseits eine weitere unkontrollierte Kollision von benachbarten Medikamentenbehältern verhindert. Beim Evakuieren und beim Anschließenden Öffnen des Kunststoffschlauchs können jedoch die Medikamentenbehälter seitlich verrutschen, was den Automatisierungsaufwand zur Weiterverarbeitung der Medikamentenbehälter erhöht. Ferner können die Medikamentenbehälter nach dem Öffnen des Kunststoffschlauchs dennoch unkontrolliert kollidieren, was die vorgenannten Nachteile mit sich bringt. Die Medikamentenbehälter können nicht in dem Transport- oder Verpackungsbehälter oder in der Haltestruktur weiterverarbeitet werden, sondern müssen zunächst in der herkömmlichen Weise vereinzelt und an nachgeordnete Bearbeitungsstationen übergeben werden.

Weitere vergleichbare Transport- und Verpackungsbehälter und Haltestrukturen werden in WO 2011/135085 A1 und WO 2009/015862 A1 offenbart. Zur Weiterverarbeitung müssen die Medikamentenbehälter jedoch stets vereinzelt werden. Eine chargenweise Weiterverarbeitung der Medikamentenbehälter, während diese in einer plattenförmigen Haltestruktur, wie vorstehend ausgeführt, aufgenommen sind, ist nicht möglich.

Die Figuren 1 bis 4 der WO 2009/015862 A1 offenbaren eine Haltestruktur, bei der elastische Haltezungen fest gegen den verengten Halsabschnitt am oberen Ende der Fläschchen drücken, um die Fläschchen reibschlüssig zu fixieren. Die Haltestruktur ist somit nur sehr eingeschränkt für Fläschchen mit hohen Toleranzen oder anderen Außendurchmessern geeignet. Ferner können die Fläschchen in der Haltestruktur nicht spannungsfrei gehalten werden, was insbesondere bei der Prozessierung, beispielsweise in einem Gefriertrockenschrank, zu einer unerwünschten Aufwölbung der Haltestruktur führen kann. Die Fläschchen können auch nicht von oben her in die Öffnungen der Haltestruktur eingeführt werden.

Bei den vorgenannten Haltestrukturen wird der Außendurchmesser der Fläschchen quasi als Hilfskontur zur Fixierung der Fläschchen an der Haltestruktur verwendet. Derartige Haltestrukturen sind deshalb nicht flexibel genug für Fläschchen mit größeren Toleranzen und/oder anderen Außendurchmessern einsetzbar.

Jedenfalls ist ein unmittelbarer Kontakt der Böden der Medikamentenbehälter, insbesondere der Böden von Fläschchen, bei den herkömmlichen Haltestrukturen nicht möglich. Dies erschwert jedoch die Weiterverarbeitung der Medikamentenbehälter insbesondere dann, wenn deren Inhalt einer Gefriertrocknung (auch als Lyophilisation oder Sublimationstrocknung bezeichnet) unterzogen werden soll. Ferner ist eine Weiterverarbeitung der Medikamentenbehälter unmittelbar in den Haltestrukturen nicht möglich, da diese dort entweder starr gehalten werden oder für die Weiterverarbeitung nicht in ausreichendem Maß zugänglich sind, weshalb die Medikamentenbehälter für eine Weiteverarbeitung herkömmlich stets aus den Haltestrukturen entnommen werden müssen, was zeitaufwendig und teuer ist. DE 10 2004 035 061 A1 offenbart ein Verfahren zum Befüllen und Wiegen von Behältern, die in einem Träger angeordnet sind, wobei die Behälter beim Wiegen angehoben und abgesenkt werden. DE 10 2004 035 061 A1 offenbart nicht den Schritt des Verschließens durch Herabdrücken der Kappen mittels Gegeneinanderdrücken von Ablageflächen. Ebenfalls wird nicht die spielbehaftete Halterung der Behälter in Trägern mit Haltezungen offenbart, die während aller genannten Bearbeitungsschritte (Befüllen, Wiegen, Verschließen) gegeben ist.

US 2009 0158612 A1 offenbart ein Verfahren zum Gefriertrocknen, bei dem eine Mehrzahl von Vials in einer Matrix-Anordnung auf einer Ablage angeordnet werden. Eine Vorrichtung, mit einer Mehrzahl von Eindringkörpern, die in der gleichen Matrix-Anordnung angeordnet sind, kann von einer ersten Stellung, in der die Eindringkörper beabstandet zu den Vials sind, in eine zweite Stellung verstellt werden, in der sich die Eindringkörper durch Verschlusskappen hindurch in die Vials hinein erstrecken. In dieser zweiten Stellung wird der eigentliche Gefriertrocknungsprozess ausgeführt. Nach einem abschließenden Druckausgleich wird die Vorrichtung zurück in die zweite Stellung verstellt. Die Vials sind während der Verarbeitung unmittelbar auf der Ablagefläche abgestützt.

### Zusammenfassung der Erfindung

Aufgabe der vorliegenden Erfindung ist es, ein einfaches und zuverlässiges Verfahren zum Verschließen von Behältern, insbesondere Fläschchen, bereitzustellen, bei dem die Behälter an einem Träger zum gleichzeitigen Halten einer Mehrzahl von Behältern in einer regelmäßigen Anordnung gehalten werden.

Diese Aufgabe wird gemäß der vorliegenden Erfindung durch ein Verfahren zum Verschließen von Behälter nach Anspruch 1 gelöst. Weitere vorteilhafte Ausführungsformen sind Gegenstand der rückbezogenen Unteransprüche.

Somit wird ein Verfahren zum Verschließen von Behältern bereitgestellt, die zur Aufbewahrung von Substanzen für medizinische, pharmazeutische oder kosmetische Anwendungen dienen oder diese enthalten, wobei die Behälter mittels einer Fördereinrichtung zur Behandlung oder Verarbeitung automatisiert an zumindest einer Prozessstation vorbeigeführt werden oder diese durchlaufen. Bei dem Verfahren werden eine Mehrzahl von Behältern, insbesondere Fläschchen (vials), von der Fördereinrichtung gefördert, während diese an einem Träger bzw. einer Haltestruktur, wie nachfolgend beispielhaft beschrieben, gemeinsam in einer regelmäßigen Anordnung gehalten werden, werden die an dem Träger gehaltenen Behälter zur Behandlung oder Verarbeitung an oder in der zumindest einen Prozessstation in eine angehobene Position angehoben und werden die Behälter nach der Behandlung oder Verarbeitung abgesenkt, um an dem Träger erneut in der regelmäßigen Anordnung gehalten zu werden.

Bei dem Verfahren umfasst die Behandlung oder Verarbeitung das Befüllen der Behälter mit der Substanz, während diese an dem Träger gehalten sind, sowie den Schritt, wonach die Behälter vor dem Befüllen und nach dem Befüllen gewogen werden, wobei das Verfahren ferner den Schritt des Verschließens der Behälter umfasst, wobei der Schritt des Verschließens der Behälter die folgenden Schritte umfasst: Aufdrücken von Kappen auf die oberen Ränder der Behälter vor oder nach dem Befüllen, und Gegeneinanderdrücken von Ablageflächen, auf denen die Böden der Behälter unmittelbar aufliegen, sodass die Kappen auf die Behälter herabgedrückt und diese somit steril verschlossen werden; wobei die Behälter in Öffnungen oder Aufnahmen des Trägers in der regelmäßigen Anordnung gehalten werden, wobei den Öffnungen oder Aufnahmen jeweils zumindest zwei elastische Haltezungen zugeordnet sind und wobei die Behälter vor dem Herabdrücken der Kappen jeweils von den Haltezungen im Bereich eines verengten Halsabschnitts unterhalb eines verbreiterten oberen Rands am oberen Endes des jeweiligen Behälters mit radialem Spiel und mit axialem Spiel oder mit radialem Spiel und axial frei verschiebbar gehalten werden

Somit kann auf die Behälter ausschließlich von deren Unterseite und/oder Seitenfläche eingewirkt werden, um eine gewünschte axiale Verstellung zur Bearbeitung oder Verarbeitung der Behälter und deren Inhalte zu gewährleisten. Somit kann die Gefahr des Eindringens von Verunreinigungen von oben her in das Innere der Behälter erfindungsgemäß minimiert werden. Da die Behälter beim Einwirken auf deren Unterseite und/oder Seitenfläche auch zuverlässiger geführt und verstellt werden können, eignet sich das erfindungsgemäße Verfahren insbesondere auch zur Ausführung in sterilen Prozessumgebungen, beispielsweise in einem Steriltunnel oder dergleichen, wo Wartungs- und Montagearbeiten einen Betrieb der Anlage unterbrechen würden und somit so weit als möglich vermieden werden können.

Weil die Behälter gemeinsam an dem Träger in einer vorbestimmten regelmäßigen Anordnung gehalten sind, ist der Aufwand zur Automatisierung des Verfahrens geringer. Insbesondere können auch eine Mehrzahl von Behältern gleichzeitig bearbeitet oder verarbeitet werden, insbesondere reihen- oder chargenweise. Zum Anheben der Behälter in die angehobene Stellung genügt ein berührender Kontakt einer Vertikal-Verstelleinrichtung mit der Unterseite und/oder Seitenfläche der Behälter, um die Behälter mechanisch zu verschieben. Das Absenken der Behälter in die Ausgangsstellung kann dabei durch Saugen, also mittels eines Unterdrucks, durch geeignetes Greifen der Behälter an deren unterem Rand, beispielsweise durch eine vorübergehende mechanisch oder pneumatisch bewirkte Klemmung der Behälter, oder durch Herabdrücken der Behälter erfolgen.

Gemäß einer weiteren Ausführungsform verhindert die Vertikal-Verstelleinrichtung eine seitliche Ausweichbewegung der Behälter zumindest beim Anheben der Behälter in die angehobene Position. Dies kann insbesondere dadurch realisiert werden, dass die Vertikal-Verstelleinrichtung zum Anheben der Behälter ausschließlich eine Bewegung exakt in vertikale Richtung ausführt (was durch eine einfache Geradführung der Vertikal-Verstelleinrichtung realisiert werden kann), dabei jedoch die Behälter so fixiert, beispielsweise durch Greifen oder Klemmen des Bodenbereichs und/oder der Seitenfläche der Behälter, dass ein Verrutschen der Behälter auf der Vertikal-Verstelleinrichtung verhindert ist.

Gemäß einer weiteren Ausführungsform umgreift die Vertikal-Verstelleinrichtung zum Anheben der Behälter einen unteren Rand der Behälter vorübergehend, was beispielsweise durch einfache mechanische Greifer oder durch eine pneumatisch betriebene Manschette bewirkt werden kann.

Gemäß einer weiteren Ausführungsform wird der untere Rand der jeweiligen Behälter während des Anhebens mittels einer Mehrzahl von Haltearmen umgriffen. Da diese Haltearme normalerweise seitlich von der Vertikal-Verstelleinrichtung abragen, sind Öffnungen oder Aufnahmen in dem Träger, in welchem die Behälter in deren Ausgangsposition an dem Träger gehalten werden und die von der Vertikal-Verstelleinrichtung beim Anheben der Behälter in die angehobene Position durchgriffen werden, bevorzugt korrespondierend ausgebildet. Insbesondere können diese korrespondierende Aussparungen aufweisen, die auch einer weiteren Zentrierung und Führung der Vertikal-Verstelleinrichtung beim Anheben der Behälter dienen können.

Gemäß einer weiteren Ausführungsform weist die die Vertikal-Verstelleinrichtung eine Mehrzahl von Hubstangen auf, die entlang einer Linie fluchtend angeordnet sind und eine Reihe von Behältern gleichzeitig in die angehobene Position verschieben können. Somit ist eine chargen- oder reihenweise Verarbeitung oder Bearbeitung der Behälter in der angehobenen Position möglich. Die Hubstangen können dabei mechanisch, pneumatisch oder hydraulisch verstellt werden. Eine so ausgelegte Vertikal-Verstelleinrichtung kann insbesondere innerhalb der Prozessstation angeordnet sein, also insbesondere auch in einer sterilen Prozessumgebung.

Gemäß einer weiteren Ausführungsform weist das Verfahren weiterhin eine Relativ-Positionierung des Trägers bzw. der Haltestruktur mit den daran gehaltenen Behältern und der Vertikal-Verstelleinrichtung auf. Hierzu erfolgt eine Relativ-Positionierung des Trägers bzw. der Haltestruktur und/oder der Vertikal-Verstelleinrichtung, beispielsweise mit Hilfe von formschlüssig zusammenwirkenden, korrespondierend zueinander ausgebildeten Elementen.

Gemäß einer weiteren Ausführungsform werden die Behälter zum Absenken mittels eines auf einen Bodenbereich der Behälter einwirkenden Unterdrucks an den Träger zurückgezogen. Somit wird während des gesamten Verfahrens ausschließlich von unten her auf die Behälter eingewirkt, was die Gefahr des Eindringens von Verunreinigungen in die Behälter weitestgehend minimiert.

Gemäß einer weiteren Ausführungsform werden die Behälter zum Absenken seitlich geklemmt, um nach der Behandlung oder Verarbeitung in die regelmäßige Anordnung an dem Träger zurückgezogen zu werden.

Gemäß einer weiteren Ausführungsform werden die Behälter zum Absenken nach unten gedrückt. Dies kann beispielsweise mit Hilfe von Greifern realisiert werden, die auf die Seitenwände oder den verschmälerten Nackenabschnitt der Behälter einwirken.

An dem Träger, der bei dem erfindungsgemäßen Verfahren eingesetzt wird und der bevorzugt flächig, insbesondere rechteckförmig ausgebildet ist, sind als Haltemittel zumindest zwei Haltezungen vorgesehen, die am Rand einer jeweiligen Öffnung oder Aufnahme vorgesehen sind und von einer Oberseite des Trägers abragen, um den jeweiligen Behälter in der Öffnung oder Aufnahme zu halten. Dabei sind die Haltezungen bevorzugt so ausgelegt, dass diese beim Einführen der Behälter in die Öffnungen oder Aufnahmen elastisch weggeschwenkt oder weggeklappt werden. Ferner sind die Haltezungen erfindungsgemäß so auf die Behälter abgestimmt, dass diese mit radialem Spiel von den Haltezungen gehalten sind. Das radiale Spiel ermöglicht, dass Behälter mit unterschiedlichen radialen Toleranzen und/oder Außenabmessungen von derselben Haltestruktur zuverlässig gehalten werden können. Das radiale Spiel ist zweckmäßig so ausgelegt und auf die Außenkontur und -abmessung der Behälter abgestimmt, dass niemals gleichzeitig sämtliche Haltezungen den verengten Halsabschnitt am oberen Ende der Behälter, insbesondere Fläschchen, berühren. Gleichzeitig verhindert das radiale Spiel auch ein unerwünschtes Verspannen oder gar Aufwölben des Trägers beim Halten von Behältern mit unterschiedlichen radialen Toleranzen und/oder Außenabmessungen, was erhebliche Vorteile insbesondere bei der gleichzeitigen Prozessierung einer Mehrzahl von Behältern, während diese von der Haltestruktur gehalten sind, bietet, beispielsweise bei der Gefriertrocknung bei Prozessierung bei sehr niedrigen Temperaturen.

Selbst wenn sich der Träger dennoch bei der Prozessierung verziehen oder aufwölben sollte, kann dennoch ein gleichmäßiger Bodenkontakt zu sämtlichen von der Haltestruktur gehaltenen Behältern realisiert werden, insbesondere wenn diese ergänzend mit einem ausreichenden axialen Spiel von den Haltezungen an der Haltestruktur gehalten sind, da das axiale Spiel zusätzlich auch einen Längentoleranzausgleich ermöglicht.

Die Haltezungen sind dabei ausreichend elastisch ausgebildet oder gelagert, sodass die Behälter axial, d.h. in Richtung der Längsachse der Behälter und senkrecht zur Ebene des Trägers, von der Ober- oder Unterseite des Trägers her in die Öffnungen oder Aufnahmen eingeschoben werden können, insbesondere unter elastischer Verformung der Haltezungen, beispielsweise unter Wegbiegen derselben. Die Bestückung des Trägers mit Behältern kann somit einfach automatisiert werden, was durch eine regelmäßige Anordnung der Öffnungen oder Aufnahmen, bevorzugt in einer zweidimensionalen Matrix, noch weiter begünstigt wird.

Erfindungsgemäß werden die Behälter von den Haltezungen an der Unterseite eines verbreiterten oberen Randabschnittes der Behälter gehalten oder abgestützt, wie dieser insbesondere bei Fläschchen typischerweise als sog. Rollrand oder Schulter vorgesehen ist. In diesem Bereich steht bei dem erfindungsgemäßen Verfahren eine Abstütz- oder Lagerfläche zum Halten oder Abstützen der Behälter mit einer ausreichenden Erstreckung in Radialrichtung der Öffnungen oder Aufnahmen zur Verfügung, um das vorgenannte radiale Spiel bei der Halterung der Behälter ohne Weiteres zu realisieren.

Weil die Behälter in den Öffnungen oder Aufnahmen erfindungsgemäß mit sehr geringem Kraftaufwand angehoben oder bewegt, beispielsweise gedreht, werden können, können diese, während diese sich in der Haltestruktur befinden und von dieser gehalten oder zumindest geführt werden, ohne weiteres bearbeitet werden. Als besonders vorteilhaft hat sich diese Art der Halterung z.B. beim Verschließen der Behälter durch Bördeln eines Metalldeckels erwiesen. Die hierzu erforderlichen Vorgänge können an dem Metalldeckel ausgeführt werden, während der Behälter in der Öffnung oder Aufnahme der Haltestruktur gehalten oder zumindest geführt ist. Als besonders vorteilhaft hat sich diese Art der Halterung auch bei der Prozessierung von Behältern erwiesen, während diese in der Haltestruktur gehalten bzw. aufgenommen sind. Beispielsweise können die Haltestrukturen mit den darin aufgenommenen bzw. gehaltenen Behältern in einen Gefriertrockenschrank eingebracht werden. Aufgrund der Halterung der Behälter mit einem gewissen Spiel in den Haltestrukturen kann gewährleistet werden, dass die Böden von sämtlichen Behältern auf einer kühlenden Unterlage, beispielsweise einem Kühlfinger des Gefriertrockenschranks, gleichmäßig aufliegen. Oder die Behälter können ohne größeren Kraftaufwand in den Öffnungen oder Aufnahmen der Haltestruktur angehoben und zur Prozessierung gehandhabt werden.

Gemäß einer weiteren Ausführungsform werden die Behälter vollständig aus Öffnungen oder Aufnahmen, die in dem Träger vorgesehen sind und die regelmäßige Anordnung vorgeben, herausgehoben, um an oder in der Prozessstation behandelt oder verarbeitet zu werden. Die Behälter sind also vollständig von den Haltemitteln der Haltestruktur freigegeben, was eine anderweitige Positionierung der Behälter bedingt, um ein seitliches Verrutschen der Behälter bei der Bearbeitung oder Verarbeitung der Behälter zu unterbinden. Diese Positionierung kann insbesondere durch geeignete Ausgestaltung der Vertikal-Verstelleinrichtung realisiert werden, beispielsweise durch Umgreifen des unteren Rands der Behälter durch Abschnitte der Vertikal-Verstelleinrichtung.

Erfindungsgemäß sind die Haltezungen als elastische Haltezungen ausgebildet und diese verfügen über eine ausreichende Elastizität, um beim Einführen der Behälter in die Öffnungen oder Aufnahmen ausreichend elastisch weggeschwenkt oder weggeklappt zu werden, um den Behältern den Weg in die Öffnungen oder Aufnahmen freizugeben. Dies lässt sich durch geeignete Dimensionierung, Materialwahl und Auslegung der Materialstärke der Haltezungen ohne weiteres erreichen. Bevorzugt sind die Haltezungen somit aus einem Kunststoff ausgebildet.

Gemäß einer Ausführungsform sind die Haltezungen elastisch gegen eine Haltestellung vorgespannt, bevorzugt mittels eines elastischen Rückstellelements, beispielsweise einer Rückstellfeder oder eines Kunststoffblättchens oder elastischen Kunststoffgebildes, das mit der zugeordneten Haltezunge geeignet zusammenwirkt und auf der Oberseite des Trägers vorgesehen oder ausgebildet ist.

Bei dem erfindungsgemäßen Verfahren sind die Haltezungen so auf die Behälter abgestimmt, dass die Behälter mit einem verbreiterten Rand, der an einem oberen Ende der Behälter ausgebildet ist, also insbesondere mit dem vorgenannten Rollrand, lose auf Oberseiten der Haltezungen aufliegen. Die Behälter können somit ohne Widerstand nach oben wieder aus den Öffnungen oder Aufnahmen entnommen werden.

Gemäß einer Ausführungsform umgreifen die Haltezungen den verbreiterten Rand dergestalt, dass die Behälter mit radialem Spiel oder mit radialem und axialem Spiel von den Haltezungen gehalten sind. Auf diese Weise können die Behälter in den Öffnungen oder Aufnahmen axial verliersicher gehalten werden. Zum Entnehmen der Behälter aus den Öffnungen oder Aufnahmen brauchen die Haltezungen nur wiederum in der Art, wie beim Einführen der Behälter, zurückgeschwenkt oder zurückgeklappt werden.

Gemäß einer Ausführungsform sind die Haltezungen so verteilt auf der Oberseite des Trägers angeordnet, dass diese beim Wegschwenken oder Wegklappen einander nicht unmittelbar berühren und eine unmittelbar benachbarte Öffnung oder Aufnahme nicht versperren. Somit kann die Packungsdichte der Behälter an dem Träger noch weiter erhöht werden. Insbesondere sind die Haltezungen so ausgelegt, dass unmittelbar benachbarte Haltezungen, wenn diese beim Einführen der Behälter in die zugeordneten Öffnungen oder Aufnahmen zum Träger hin geschwenkt oder geklappt werden, einander nicht berühren.

Gemäß einer Ausführungsform sind am oberen Ende der Haltezungen Einführschrägen ausgebildet, welche jeweils in eine von den Haltezungen radial einwärts vorstehende Haltenase zum Halten der Behälter übergehen. Die Behälter können somit noch einfacher und kraftärmer in die Öffnungen oder Aufnahmen eingeführt werden. Insbesondere geraten beim Einführen der Behälter von oben her in die Öffnungen oder Aufnahmen zunächst die Böden bzw. unteren Enden der Behälter in Anlage zu den Einführschrägen. Beim weiteren Einführen der Behälter gleitet das untere Ende bzw. der Boden der Behälter entlang den Einführschrägen abwärts und spreizt dabei die Haltezungen auseinander oder klappt bzw. schwenkt diese zurück. Beim weiteren Einführen der Behälter gerät schließlich die zylindrische Seitenwand in Anlage zu den Haltenasen und gleitet an diesen entlang, solange bis schließlich die Unterseite des vorgenannten Rollrands lose auf den Haltenasen der Haltezungen aufliegt.

Gemäß einer Ausführungsform sind die einer jeweiligen Öffnung oder Aufnahme zugeordneten Haltezungen oder deren Einführschrägen gleichsinnig und um einen Winkel kleiner 90° verdrillt ausgebildet, sodass die Haltezungen beim Einführen der Behälter von der Oberseite des Trägers her in die Öffnungen oder Aufnahmen, in Draufsicht betrachtet, radial und mit einer Bewegungskomponente in Umfangsrichtung weggeschwenkt oder weggeklappt werden. Dies kann je nach der Anordnung und Verteilung der Haltezungen auf dem Träger ermöglichen, dass unmittelbar benachbarte Haltezungen, wenn diese beim Einführen der Behälter in die zugeordneten Öffnungen oder Aufnahmen zum Träger hin geschwenkt oder geklappt werden, einander nicht berühren.

Gemäß einer weiteren Ausführungsform sind die Öffnungen oder Aufnahmen auf einer der Oberseite gegenüberliegenden Unterseite des Trägers zumindest abschnittsweise von einer jeweiligen Seitenwand begrenzt, um eine Berührung von Behältern in unmittelbar benachbarten Öffnungen oder Aufnahmen zu verhindern, wobei die Seitenwände ganz besonders bevorzugt so ausgebildet sind, dass die Behälter von der Unterseite des Trägers her frei zugänglich sind. Die Seitenwände von benachbarten Öffnungen oder Aufnahmen sind bevorzugt miteinander verbunden, was vorteilhaft zu einer weiteren Versteifung des Trägers beiträgt. Bevorzugt sind die Seitenwände einstückig mit dem Träger ausgebildet, was beispielsweise in Kunststoff-Spritzgusstechnik einfach realisiert werden kann.

Die Böden bzw. unteren Enden der in den Öffnungen oder Aufnahmen aufgenommenen Behälter stehen bevorzugt von den unteren Enden der Seitenwände vor, sodass die Böden der Behälter von der Unterseite des Trägers her frei zugänglich sind. Dies ermöglicht, dass die Behälter prozessiert werden können, während diese an dem Träger in den Öffnungen oder Aufnahmen gehalten sind, wie weiter unten ausgeführt.

Gemäß einer weiteren Ausführungsform sind die Haltezungen mit dem Träger einstückig ausgebildet, was eine kostengünstige Herstellung ermöglicht, beispielsweise durch Spritzgießen aus einem Kunststoff. Die elastischen Haltezungen ragen dabei bogenförmig von der Oberseite des Trägers ab und, in Draufsicht betrachtet, bevorzugt ein wenig in die zugeordnete Öffnung oder Aufnahme hinein. So können die Behälter insbesondere im Bereich eines verengten Halsabschnitts und nahe dem oberen offenen Ende eines Behälters bzw. Fläschchens gehalten werden, wie nachfolgend näher ausgeführt. Die bogenförmige Ausbildung der Haltezungen erleichtert das Einschieben und wieder Herausziehen der Behälter in die bzw. aus den Öffnungen oder Aufnahmen des Trägers.

Gemäß einer weiteren Ausführungsform sind die einer Öffnung bzw. Aufnahme zugeordneten elastischen Haltezungen jeweils bezüglich einer Mittellinie der Öffnung oder Aufnahme symmetrisch angeordnet und ausgebildet. Die Behälter werden somit automatisch zentriert in den jeweiligen Öffnungen oder Aufnahmen des Trägers gehalten. Die Symmetrie verhindert auch ein versehentliches Verkippen oder Verkanten der Behälter beim Einführen oder Halten in den Öffnungen oder Aufnahmen des Trägers.

Gemäß einer weiteren Ausführungsform bilden die elastischen Haltezungen jeweils eine Dreipunkt-Lagerung zum Halten der Behälter in der jeweiligen Öffnung oder Aufnahme des Trägers aus, wodurch eine automatische Zentrierung der Behälter in den zugeordneten Öffnungen oder Aufnahmen und eine sehr präzise und stabile Festlegung der Position der Behälter an dem Träger noch mehr begünstigt ist.

Gemäß einer weiteren Ausführungsform sind die Öffnungen oder Aufnahmen in einer regelmäßigen Anordnung von Reihen und Spalten auf dem Träger verteilt angeordnet, wobei die jeweils Reihen und Spalten jeweils regelmäßig versetzt zueinander angeordnet sind und eine wiederkehrende Anordnung ausbilden. Diese regelmäßige Anordnung ist für eine automatisierte Behandlung der Behälter vorteilhaft.

Gemäß einer weiteren Ausführungsform ist die Wiegeeinrichtung bevorzugt in eine zum Anheben und Absenken der Behälter verwendete Vertikal-Verstelleinrichtung integriert, wie nachfolgend beschrieben.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a und 1b: in einer perspektivischen Draufsicht und einer Draufsicht eine Haltestruktur gemäß einer ersten Ausführungsform zur Verwendung bei einem Verfahren gemäß der vorliegenden Erfindung;
- Fig. 1c: einen Teilschnitt der Haltestruktur gemäß A-A von Fig. 1b;
- Fig. 1d: einen stark vergrößerten Teilschnitt in dem Einsatz, der in der Fig. 1c dargestellt ist;
- Fig. 1e: in dem stark vergrößerten Teilschnitt nach der Fig. 1d die Halterung eines Behälters in einer der Öffnungen einer Haltestruktur gemäß der der ersten Ausführungsform;
- Fig. 2a: in einem perspektivischen Teilschnitt und in Draufsicht einen Transport- oder Verpackungsbehälter mit einer darin aufgenommenen Haltestruktur gemäß einer zweiten Ausführungsform zur Verwendung bei einem Verfahren gemäß der vorliegenden Erfindung und mit von dieser gehaltenen Behältern;
- Fig. 2b: den Transport- oder Verpackungsbehälter gemäß der Fig. 2a in einem Teilschnitt und in Draufsicht;
- Fig. 2c: in zwei vergrößerten Teilschnitten die Halterung von Behältern in der Haltestruktur gemäß der zweiten Ausführungsform sowie Details davon;
- Fig. 2d: in einer perspektivischen Draufsicht die Haltestruktur gemäß der Fig. 2a ohne Behälter;
- Fig. 2e: in einer perspektivischen Unteransicht die Haltestruktur gemäß der Fig. 2a ohne Behälter;
- Fig. 2f: einen weiteren Teilschnitt der Haltestruktur gemäß der Fig. 2a ohne Behälter;
- Fig. 2g: in einem stark vergrößerten Teilschnitt die Halterung eines Behälters in einer Haltestruktur gemäß einer weiteren Ausführungsform der vorliegenden Erfindung;
- Fig. 2h: eine stark vergrößerte Draufsicht auf eine Einführschräge einer Haltezunge gemäß einer Variante zu der Haltestruktur gemäß der Fig. 2a;
- Fig. 2i: eine weitere Variante von Haltezungen für eine Haltestruktur gemäß der Fig. 2a;
- Fig. 2j: einen Teilschnitt durch eine weitere Variante einer Haltestruktur gemäß der Fig. 2a mit darin gehaltenem Behälter;
- Fig. 2k und 2l: weitere Varianten einer Haltestruktur zur Verwendung bei einem Verfahren gemäß der vorliegenden Erfindung;
- Fig. 2m bis 2o: in einer Bewegungssequenz das Einführen eines Behälters in eine Haltestruktur gemäß einer weiteren Variante;
- Fig. 3a bis 3c: eine Haltestruktur gemäß einer weiteren Ausführungsform zur Verwendung bei einem Verfahren gemäß der vorliegenden Erfindung in einer perspektivischen Draufsicht, in einer Draufsicht und in einer Schnittansicht;
- Fig. 3d: einen Teilschnitt durch die Haltestruktur gemäß der Fig. 3a;
- Fig. 3e: in einer stark vergrößerten Teildraufsicht die Verhakung von ineinander eingreifenden Vorsprüngen und Aussparungen an den Rändern zweier Haltestrukturen gemäß der Fig. 3a;
- Fig. 3f: einen Querschnitt entlang A-A gemäß der Fig. 3e;
- Fig. 4a: einen Ausschnitt aus einer Bearbeitungsstation zur Bearbeitung oder Prozessierung von Fläschchen gemäß der vorliegenden Erfindung, während die Fläschchen in einer angehobenen Stellung in einer Haltestruktur gemäß der Fig. 2d gehalten sind;
- Fig. 4b bis 4g: in schematischen Seitenansichten oder Teilschnitten eine Sequenz von Prozessschritten bei einem Verfahren gemäß der vorliegenden Erfindung in der Bearbeitungsstation gemäß der Fig. 4a, wobei Behälter ausgehend von einer Ausgangsstellung (Fig. 4b) in eine angehobene Stellung überführt werden (Fig. 4g);
- Fig. 5a bis 5f: eine entsprechende Sequenz von Prozessschritten gemäß einer weiteren Variante des Verfahrens gemäß der vorliegenden Erfindung;
- Fig. 6a: in einer Seitenansicht das Zusammenwirken eines Stößels der Bearbeitungsstation gemäß der Fig. 4a und eines von diesem gehaltenen Fläschchens;
- Fig. 6b: den Stößel gemäß der Fig. 6a vor seinem Eingriff mit dem Fläschchen, wobei der in dem Stößel geführte Unterdrucksauger sichtbar ist;
- Fig. 6c: einen Querschnitt entlang B-B gemäß der Fig. 6b;
- Fig. 7a: eine Explosionsansicht des Stößels gemäß der Fig. 6b und des Unterdrucksaugers;
- Fig. 7b bis 7d: verschiedene Detailansichten des Stößels gemäß der Fig. 7a;
- Fig. 8a: eine Lochplatte mit Führungsstruktur zum Führen und Positionieren des Stößels gemäß der Fig. 7a in einer Perspektivansicht;
- Fig. 8b: eine Draufsicht auf die Lochplatte mit Führungsstruktur gemäß der Fig. 8a;
- Fig. 8c: eine Detailansicht aus der Draufsicht gemäß der Fig. 8b;
- Fig. 8d: eine Schnittansicht entlang B-B gemäß der Fig. 8b; und
- Fig. 9a: ein schematisches Flussdiagramm eines Verfahrens zur Prozessierung oder Bearbeitung von Fläschchen mittels einer Bearbeitungsstation gemäß der Fig. 4a;
- Fig. 9b und 9c: Beispiele für die Verwendung eines solchen Verfahrens zum Bördeln oder Crimpen eines Metalldeckels auf einem oberen Rand von Fläschchen;
- Fig. 9d: ein schematisches Flussdiagramm eines weiteren Verfahrens zur Prozessierung oder Bearbeitung von Fläschchen mittels einer Bearbeitungsstation gemäß der Fig. 4a;
- Fig. 9e und 9f: ein weiteres Beispiel für die Verwendung eines solchen Verfahrens zum Verschließen von Fläschchen mit Hilfe von steril verschließenden Kappen, die unmittelbar auf den oberen Rand der Fläschchen aufgedrückt werden; und
- Fig. 10a und 10b: Einzelheiten einer Vorrichtung gemäß der vorliegenden Erfindung zum Wiegen eines Behälters, während dieser in einer Haltestruktur aufgenommen oder geführt ist.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Eine Haltestruktur sowie ein Transport- und Verpackungsbehälter, der eine solche Haltestruktur aufnimmt, dienen gemäß der vorliegenden Erfindung, wie nachfolgend beschrieben, der gleichzeitigen Halterung einer Mehrzahl von Behältern zur Aufbewahrung von Substanzen für kosmetische, medizinische oder pharmazeutische Anwendungen, und zwar bevorzugt in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen oder in regelmäßigen Reihen, die relativ zueinander versetzt angeordnet sind. Nachfolgend wird zunächst die Haltestruktur beschrieben, bevor das Verfahren und eine Vorrichtung gemäß der vorliegenden Erfindung beschrieben werden.

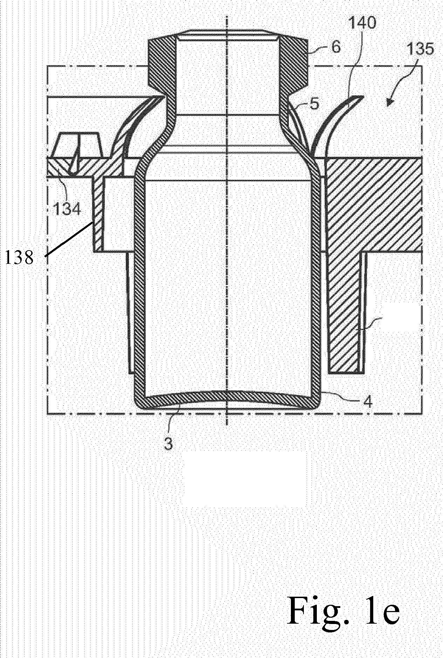

Ein Beispiel für derartige Medikamentenbehälter in Gestalt von Fläschchen ist in der Fig. 1e schematisch in einem Längsschnitt dargestellt. Diese weisen eine zylindrische Grundform auf, mit einer zylinderförmigen Seitenwand mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 (auch Rollrand) übergeht, der einen größeren Außendurchmesser aufweist als der zugeordnete Halsabschnitt 5 und zur Verbindung mit einem Verschlusselement ausgelegt ist. Wie man der Fig. 1e entnehmen kann, ist die Unterseite des Rollrands 6 abgeschrägt ausgebildet und erstreckt sich unter einem spitzen Winkel abwärts und hin zu dem verengten Halsabschnitt 5. Wie in der Fig. 1e dargestellt, ist in Luftspalt in radialer Richtung zwischen beispielsweise der linken Haltezunge 140 (oder mehreren oder allen Haltezungen einer Öffnung oder Aufnahme) und dem verengten Halsabschnitt 5 des Behälters ausgebildet.

Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Crimpen oder Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpulen oder Spritzen- oder Injektionsbehältnisse. Ampullen oder Karpulen sind Behältnisse für Arzneimittel zur meist parenteralen Applikation (Injektion), für Kosmetika und andere Substanzen und sind meist zylindrisch geformt mit einer ausgezogenen Spitze (Spieß oder Kopf) und einem flachen Boden oder auch mit zwei ausgezogenen Spitzen an beiden Enden. Diese können insbesondere als Brechampullen mit einer ringförmigen Solbruchstelle um den Ampullenhals herum oder als OPC-Ampulle (One-Point-Cut-Ampulle) mit einem in das Glas geritzten Brechring ausgebildet sein. Spritzen- bzw. Injektionsbehältnisse, auch als Injektionsfläschchen, Stechampulle oder Mehrwegampulle bezeichnet, sind zylindrische, flaschenähnlich geformte Behältnisse aus Glas oder Kunststoff, meist in relativ kleinen Nennvolumina (z. B. 1 ml, 10 ml). Sie sind mit einem Gummistopfen mit Septum (Durchstichgummi) verschlossen. Zum Schutz des Septums und Fixierung des Gummistopfens ist noch ein äußerer Verschluss (Bördelkappe oder Krampe), oft aus Aluminiumblech, aufgebracht. Bei einer Karpule befindet sich die Flüssigkeit in einem Zylinder, der am einen Ende mit einem dicken Gummi- oder Kunststoffstopfen verschlossen ist. Dieser fungiert als Kolben, wenn der Inhalt mit einer Karpulenspritze ausgepresst wird. Am anderen Ende ist der Zylinder nur mit einer dünnen Membran verschlossen, die bei der Anwendung vom hinteren Ende der Karpulenkanüle (eine beidseitig angeschliffene Kanüle) durchstochen wird. Zylinderampullen werden häufig in der Zahnmedizin zur Lokalanästhesie verwendet. Spezielle Zylinderampullen mit besonders gestaltetem Vorderteil (z. B. Gewinde) werden zur Insulintherapie in Insulinpens verwendet.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasbehältern einen oder mehrere Zehntel Millimeter betragen können. Um solche Fertigungstoleranzen kompensieren zu können und gleichzeitig zu gewährleisten, dass sämtliche Flaschenböden 3 in einer Ebene angeordnet werden können, werden die Behälter erfindungsgemäß an einer Halterungsstruktur fixiert. Die Halterung der Behälter wird dabei im Übergangsbereich des verengten Halsabschnitts 5 zum verbreiterten oberen Rand 6 realisiert. Insbesondere liegt die Unterseite des Rands 6 der Behälter im Übergangsbereich zum verengten Halsabschnitt 5 auf den oberen Enden von Haltezungen 140 auf, wie nachfolgend näher beschrieben. Die Haltezungen 140 sind bevorzugt aus einem ausreichend flexiblen oder elastischen Kunststoff ausgebildet. Alternativ können die Haltezungen auch relativ steif ausgebildet sein, jedoch so beweglich an der Oberseite des Trägers 134 gelagert sein, dass diese beim Einführen der Behälter elastisch aus der Öffnung 135 weggeschwenkt oder zurückgeklappt werden, wie nachfolgend beschrieben. Zu diesem Zweck können die Haltezungen mittels elastischer Rückstellelemente (nicht gezeigt), beispielsweise Rückstellfedern oder elastischen Kunststoffgebilden oder -blättchen, in die in der Fig. 1e dargestellte Haltestellung elastisch vorgespannt werden.

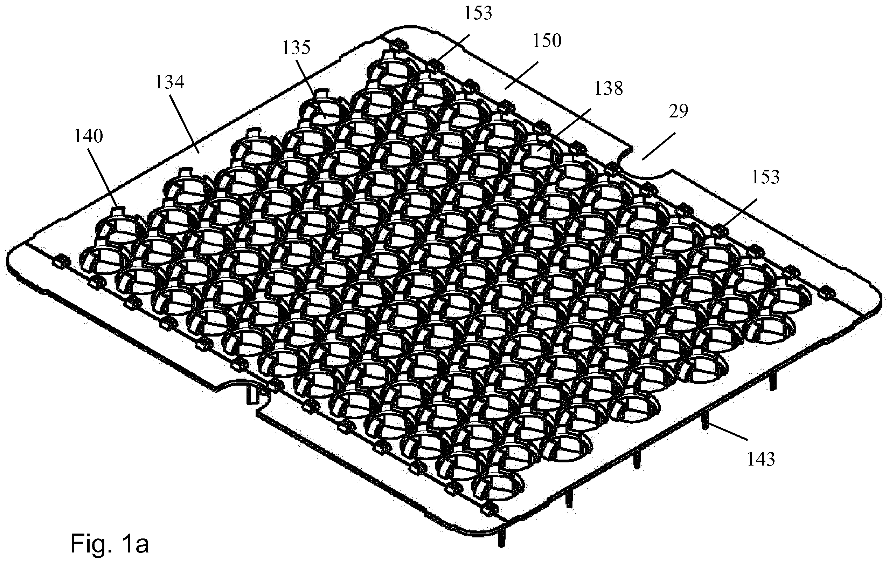

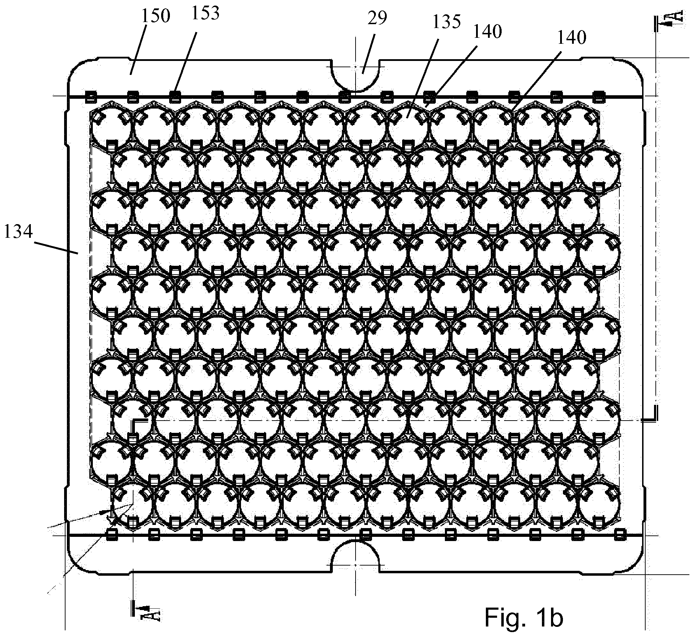

Zum gleichzeitigen Halten einer Mehrzahl von Behältern wird gemäß einer ersten Ausführungsform, wie in den Fig. 1a und 1b dargestellt, ein flächiger rechteckförmige Träger 134 bereitgestellt, der aus einem Kunststoff ausgebildet ist, beispielsweise ausgestanzt oder spritzgegossen ist, und eine Mehrzahl von Öffnungen 135 zur Aufnahme der Glasfläschchen 2 aufweist. Die Öffnungen 135 sind in einer regelmäßigen zweidimensionalen Anordnung angeordnet, bei dem dargestellten Ausführungsbeispiel in einer Matrix-Anordnung aus Reihen und sich rechtwinklig dazu erstreckenden Spalten, die unter gleichen Abständen zueinander und regemäßig zueinander versetzt in einer wiederkehrenden Anordnung angeordnet sind.

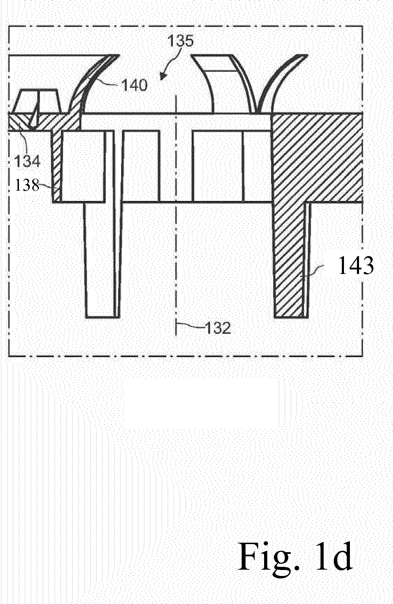

Die Öffnungen 135 sind von Seitenwänden 138 (vgl. Fig. 1d) auf der Unterseite des Trägers 134 begrenzt, um eine Kollision von Behältern, die in unmittelbar benachbarten Öffnungen 135 aufgenommen sind, zu verhindern. Gemäß der Fig. 1b ragen elastische Haltezungen 140 von der Oberseite des Trägers 134 bogenförmig ab und, in Draufsicht betrachtet, in die zugeordneten Öffnungen 135 hinein. Die elastischen Haltezungen 140 und die Seitenwände 138 sind bevorzugt einstückig mit dem flächigen Träger 134 ausgebildet sind, beispielsweise durch 1K- oder 2K-Kunststoff-Spritzgussverfahren.

Gemäß der Fig. 1b laufen die Seitenwände 138 jeweils in den Eckbereichen der Öffnungen 135 zusammen und sind dort miteinander verbunden oder einstückig ausgebildet. Von diesen Eckbereichen ragen die elastischen Haltezungen 140 in einer Anordnung mit dreizähliger Punktsymmetrie in die benachbarten Öffnungen 135 ab. Dies führt zu einer symmetrischen Kraftableitung beim Halten der Behälter über die Haltezungen 140. Die Haltezungen 140 bewirken so eine vorteilhafte Dreipunkt-Lagerung der Behälter in den Öffnungen, sodass die Behälter automatisch zentriert bezüglich einer Mittellinie 132 (vgl. Fig. 1d) einer jeweiligen Öffnung 135 gehalten werden.

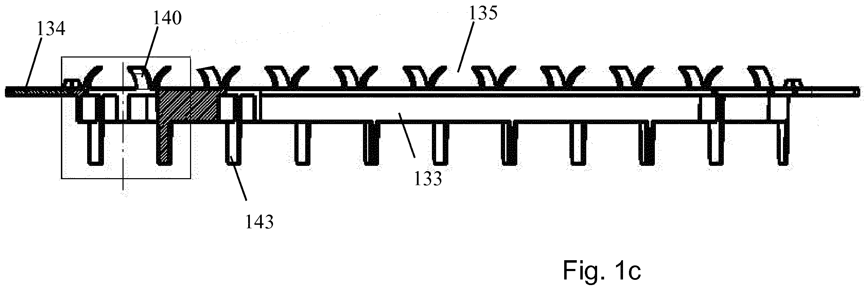

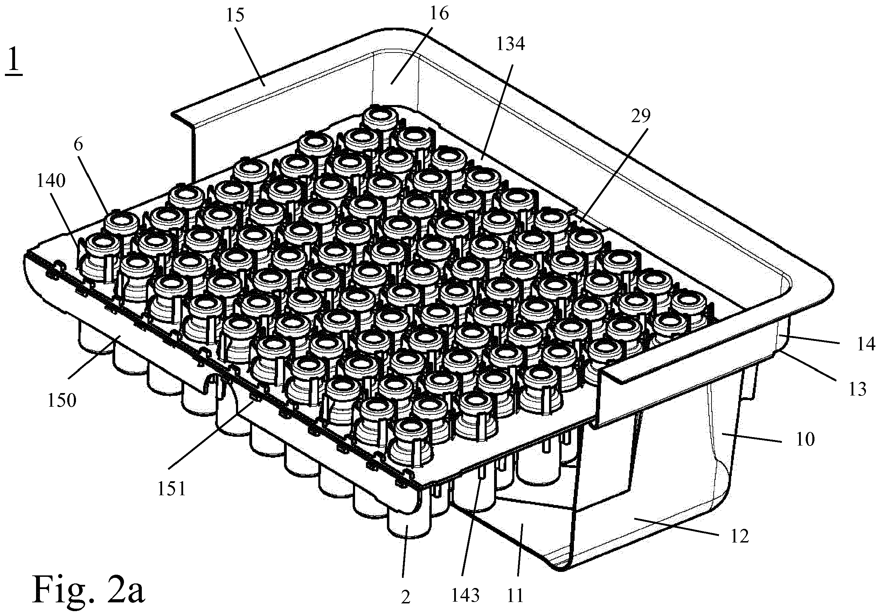

Die Fig. 1c zeigt einen Teilschnitt der Haltestruktur gemäß A-A von Fig. 1b. Erkennbar ist, dass der Träger 134 auf der Unterseite von einem umlaufenden Rand 133 begrenzt ist, auf welchem der Träger 134 auf einer umlaufenden Stufe 13 (vgl. Fig. 2a) eines Transport- oder Verpackungsbehälter 1 abgestützt werden kann.

Die Fig. 1d zeigt einen stark vergrößerten Teilschnitt in dem Einsatz, der in der Fig. 1c dargestellt ist. Erkennbar ist, dass die Behälter ohne weiteres von unten her in die Öffnungen 135 des Trägers 134 eingeführt werden können. Beim Einführen der Behälter in die Öffnungen 135 kommt es zu einem elastischen Verbiegen der elastischen Haltezungen 140.

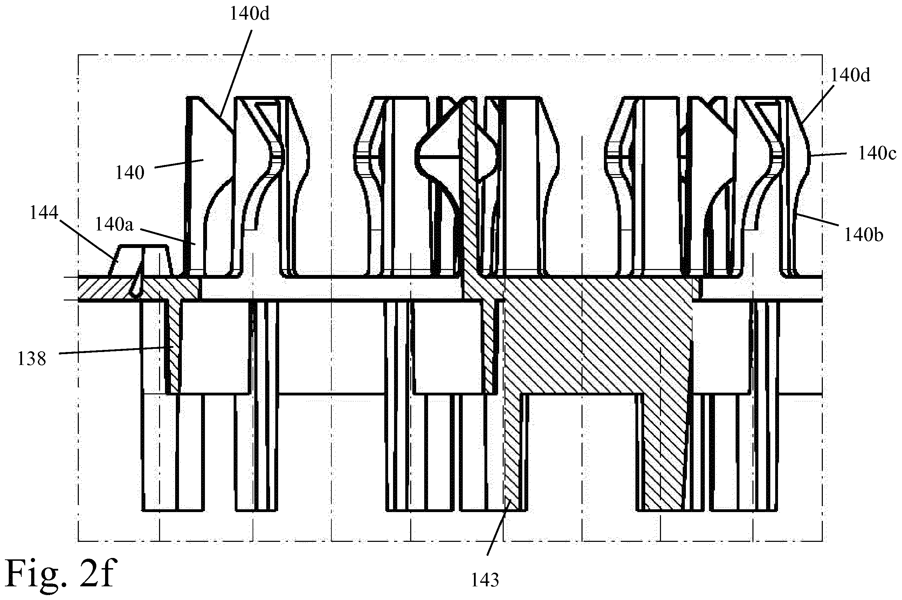

Je nach konkreter Ausgestaltung der zu haltenden Behälter können diese grundsätzlich auch von oben her in die Öffnungen 135 des Trägers 134 eingeführt werden, um an dem Träger 134 gehalten zu werden. Dies hat den Vorteil, dass das Risiko, dass Flüssigkeit oder anderer Behälterinhalt aus dem Behälterinnenraum der noch unverschlossenen Behälter unkontrolliert beim Einführen in die Öffnungen und beim Wegschwenken der Haltezungen 140 auf die Haltestruktur, insbesondere die Trägerplatte 134, gelangen können, noch weiter verringert werden kann. Zu diesem Zweck können auf der Oberseite der elastischen Haltezungen 140 Einführschrägen vorgesehen sein, wie diese nachfolgend näher anhand der Fig. 2f für eine alternative Ausführungsform beschrieben werden.

Durch Stärke, Material und Auslegung der elastischen Haltezungen 140 kann die zum Einführen und Entnehmen eines Behälters notwendige Kraft einfach vorgegeben werden.

Gemäß der vorliegenden Erfindung sind die Behälter zumindest mit radialem Spiel und bevorzugt sowohl mit radialem als auch mit axialem Spiel lose auf den Haltezungen abgestützt. Auf diese Weise können auch große Toleranzen von Behältern und unterschiedliche Außendurchmesser im Bereich des Halsabschnitts 5 einfach ausgeglichen werden. Denn zur Halterung der Behälter genügt es, wenn der Rollrand 6 noch auf den Oberseiten der Haltezungen 140 aufliegt. Grundsätzlich können dadurch auch Behälter unterschiedlichen Typs, beispielsweise mit unterschiedlichen Durchmessern im Bereich des Halsabschnitts 5, von derselben Haltestruktur gehalten werden.

Die Fig. 1e verdeutlicht dies in dem gleichen stark vergrößerten Teilschnitt wie nach der Fig. 1d und stellt die Halterung eines Behälters in einer Öffnung 135 des Träges 134 dar. Gemäß der Fig. 1e liegt die Unterseite des verbreiterten Rands 6 auf dem vorderen Ende der elastischen Haltezungen 140 im Übergangsbereich zwischen dem verengten Halsabschnitt 5 und dem Rand 6 lose auf, um die Lage des Behälters zu fixieren. Wie man in der Fig. 1e erkennen kann, besteht zwischen den Haltezungen 140 (vgl. linker Bildteil) und dem verengten Halsabschnitt 5 ein Luftspalt, der ein radiales Spiel ermöglicht. Aufgrund dieser Halterung mit radialem Spiel besteht dabei, je nach konkreter Ausbildung des Behälters, noch die Möglichkeit, den von den Haltezungen 140 gehaltenen Behälter axial zu verschieben, d.h. in Längsrichtung der Behälter, beispielsweise solange, bis die Böden 3 von allen von dem Träger 134 gehaltenen Behältern unter dem gleichen Abstand zum Träger 134 gehalten werden, um gemeinsam eine Ebene aufzuspannen.

Gemäß der Fig. 1e ist der Behälter soweit in die Öffnung 135 eingeschoben, dass der verbreiterte Rand 6 auf den vorderen Enden der Haltezungen genau an dem Übergangs bereich zwischen dem verengten Halsabschnitt 5 und dem verbreiterten oberen Rand 6 abgestützt ist. Dies lässt sich beispielsweise durch Einschieben der Behälter von unten her in die Öffnungen 135 des Trägers 134 und anschließendes Herabdrücken der Behälter bewerkstelligen, und zwar solange, bis die vorderen Enden der Haltezungen genau an dem Übergangsbereich zwischen dem verengten Halsabschnitt 5 und dem verbreiterten oberen Rand 6 anliegen. In der Haltestellung gemäß der Fig. 1e ist jedenfalls bei der großen Mehrzahl der fixierten Behälter ein gewisser radialer Abstand zwischen dem stufenartigen Übergangsbereich zwischen dem oberen Rand 6 und dem verengten Halsabschnitt 5 und vorderen Ende der Haltezungen 140 vorgesehen. Auf diese Weise können in einem gewissen Umfang Fertigungstoleranzen der Behälter in axialer Richtung und auch Fertigungstoleranzen in radialer Richtung kompensiert werden und somit auch Behälter mit unterschiedlichen Durchmessern im Bereich des verengten Halsabschnitts 5 von ein- und demselben Träger 134 gehalten werden. Damit lassen sich auch etwaige Verspannungen im Kunststoff des Trägers 134 aufgrund der Aufnahme von Behältern mit einem zu großen Außendurchmesser gering halten.

Gemäß alternativen Ausführungsformen, wie nachfolgend beschrieben, können die Behälter auch form- oder reibschlüssig an dem Träger 134 gehalten werden.

Zum Transport und zur Verpackung der vorstehend beschriebenen Haltestruktur mit den darin aufgenommenen Behältern dient ein Transport- und Verpackungsbehälter 10, wie dieser schematisch in der Fig. 2a für eine Haltestruktur bzw. einen Träger 134 gemäß einer zweiten Ausuhrungsform der vorliegenden Erfindung dargestellt ist.

Wie nachfolgend näher erläutert, können erfindungsgemäß die Behälter 2 auch innerhalb der Haltestruktur 134 oder des Transport- und Verpackungsbehälters 10 gemeinsam weiterverarbeitet werden, insbesondere auch in einem Steriltunnel oder einem Gefriertrockens chrank.

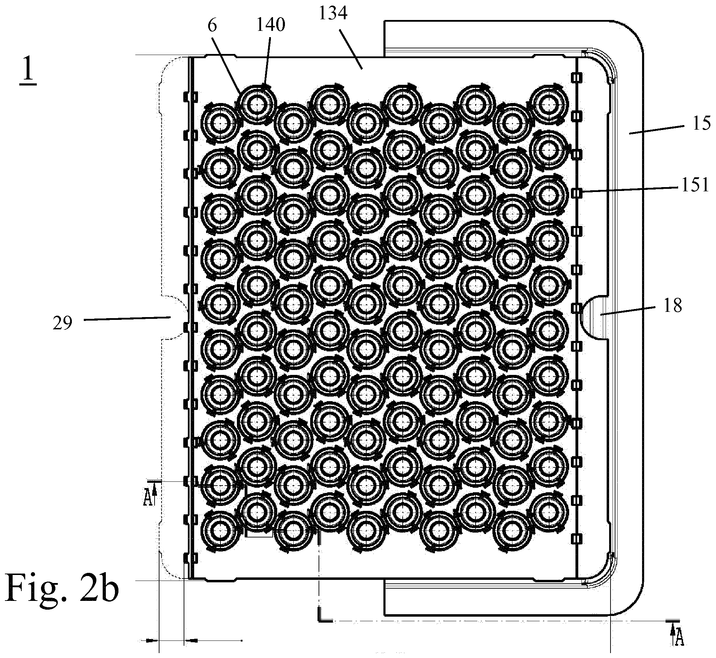

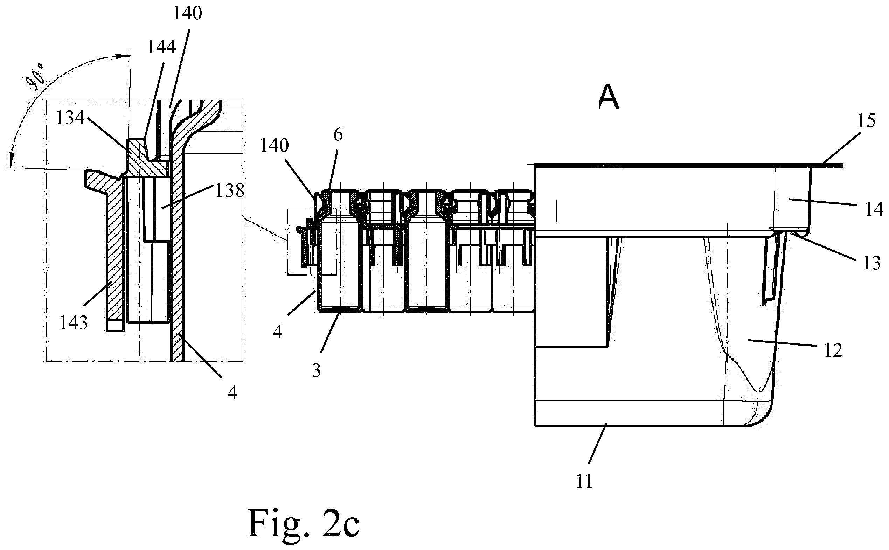

Die Fig. 2c zeigt in zwei vergrößerten Teilschnitten entlang A-A gemäß der Fig. 2b die Halterung von Behältern in der Haltestruktur gemäß der zweiten Ausuhrungsform sowie Details davon. Erkennbar ist insbesondere, dass auf der Oberseite des Trägers abgeschrägte Anschlagnasen 144 vorgesehen sind, welche das Zurückschwenken der elastischen Haltezungen 140 beim Einführen der Behälter begrenzen.

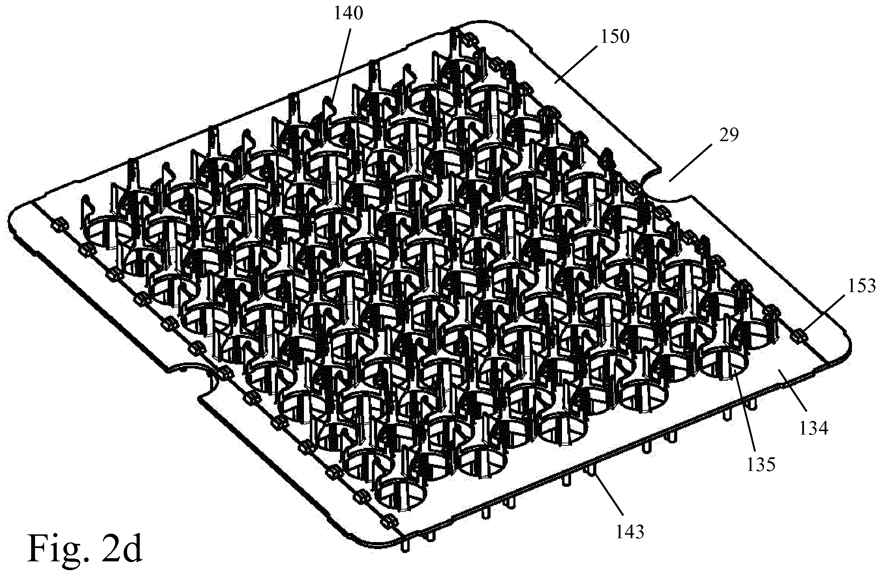

Die Fig. 2d zeigt in einer perspektivischen Draufsicht die Haltestruktur gemäß der Fig. 2a ohne Behälter. Erkennbar sind die elastischen Haltezungen 140 fähnchenartig und mit einer radial einwärts vorstehenden Haltenase ausgebildet, wie in dem stark vergrößerten Teilschnitt durch diese Haltestruktur gemäß der Fig. 2f besser dargestellt. Gemäß der Fig. 2f sind die elastischen Haltezungen 140 über eine senkrecht von der Oberseite des Trägers 134 abragende, elastische Basis 140a mit dem Träger 134 verbunden. Die Basis 140a geht über in einen radial einwärts gekrümmten Abschnitt 140b über, der schließlich in die Haltenase 140c übergeht, auf welcher der verbreiterte Rand 6 (vgl. Fig. 1e) der Behälter aufliegt, wie vorstehend anhand der Fig. 1e für die erste Ausführungsform beschrieben. Die Haltenase 140c ragt dabei in die Öffnung des Trägers 134 hinein. Die Haltenase 140c geht über in eine sich schräg aufwärts erstreckende Einführschräge 140d über, die mit dem oberen Ende der Haltezunge 140 verbindet. Aufgrund der Einführschräge 140d auf der Oberseite der Haltezunge 140 sowie des nach unten geöffneten, gekrümmten Abschnitts 140b der Haltezunge 140 können die Behälter wahlweise von oben oder von unten her in die Öffnungen des Trägers 134 eingeführt und aus diesen wieder abgezogen werden.

Beim Einführen der Behälter von oben her in die Öffnungen geraten zunächst die Böden bzw. unteren Enden der Behälter in Anlage zu den Einführschrägen 140d der Haltezungen 140. Beim weiteren Einführen der Behälter gleitet das untere Ende bzw. der Boden der Behälter entlang den Einführschrägen 140d abwärts und spreizt dabei die Haltezungen 140 zunehmend elastisch auseinander oder klappt bzw. schwenkt diese zurück. Beim weiteren Einführen der Behälter gerät schließlich die zylindrische Seitenwand der Behälter (vgl. Fig. 1e) in Anlage zu den Haltenasen 140c und gleitet an diesen entlang, solange bis schließlich die Unterseite des verbreiterten Rands der Behälter lose auf den Haltenasen 140c der Haltezungen 140 aufliegt. Die Behälter können dann entweder nach oben hin mit umgekehrtem Bewegungsablauf der Haltezungen 140 und ohne elastisches Verbiegen der Haltezungen 140 oder nach unten hin mit elastischem Verbiegen der Haltezungen 140 aus den Öffnungen des Trägers 134 entnommen werden.

Beim Einführen der Behälter von unten her in die Öffnungen gerät das obere Ende der Behälter zunächst in Anlage zu dem gekrümmten Abschnitt 140b der Haltezungen. Beim weiteren Einführen der Behälter gleitet das obere Ende der Behälter entlang den gekrümmten Abschnitten 140b aufwärts und spreizt dabei die Haltezungen 140 zunehmend elastisch auseinander oder klappt bzw. schwenkt diese zurück, bis schließlich die Haltenasen 140c erreicht sind. Beim weiteren Hochschieben der Behälter gleitet die Unterseite des verbreiterten Rands der Behälter über die Haltenasen 140c der Haltezungen 140 und liegt schließlich lose auf den Haltenasen 140c der Haltezungen 140 auf. Die Behälter können dann entweder nach unten hin mit umgekehrtem Bewegungsablauf der Haltezungen 140 und mit elastischem Verbiegen der Haltezungen 140 oder nach oben hin ohne elastisches Verbiegen der Haltezungen 140 aus den Öffnungen des Trägers 134 entnommen werden.

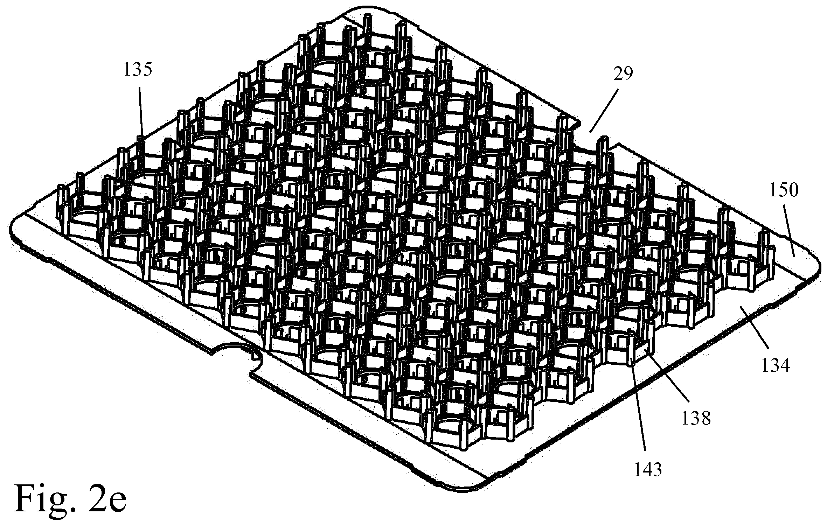

Die Fig. 2e zeigt in einer perspektivischen Unteransicht die Haltestruktur gemäß der Fig. 2a ohne Behälter. Erkennbar ist die wabenförmige, hexagonale Anordnung der umlaufenden Seitenwände 138, in deren Eckbereichen Zapfen 143 senkrecht von der Unterseite des Trägers 134 abragen. Diese Zapfen 143 dienen als Abstandshalter beim Ablegen des Trägers 134 auf einer Ablagefläche, beispielsweise dem Boden 11 eines Transport- und Verpackungsbehälters (vgl. Fig. 2a), vermeiden aber gleichzeitig auch den Kontakt der Behälter untereinander.

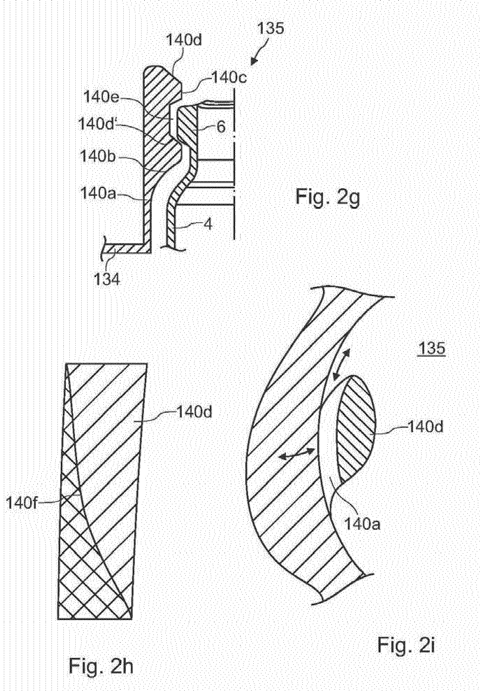

Die Fig. 2g zeigt in einem stark vergrößerten Teilschnitt die Halterung eines Behälters in einer Haltestruktur gemäß einer weiteren Ausführungsform der vorliegenden Erfindung. Abweichend zur zweiten Ausführungsform werden die Behälter hier an ihrem verbreiterten oberen Randabschnitt 6 (Rollrand) formschlüssig umgriffen, wobei ein ausreichendes radiales Spiel, wie vorstehend beschrieben, gewährleistet ist, wie durch den Luftspalt in Radialrichtung in der Fig. 2g angedeutet. Alternativ kann zusätzlich zu diesem radialen Spiel auch ein ausreichendes axiales Spiel gewährleistet sein, wie durch den Luftspalt in axialer Richtung in der Fig. 2g angedeutet. Zu diesem Zweck ist am vorderen Ende der Haltenase 140c (vgl. Fig. 2f) eine C-förmige Aussparung 140e vorgesehen, die über Schrägen 140d' in die Haltenase 140c übergeht. In der Haltestellung gemäß der Fig. 2g liegt der verbreiterte Randabschnitt 6 lose und mit radialem Spiel auf der unteren Schräge 140d' der Aussparung 140e auf. Wie in der Fig. 2g dargestellt, kann zwischen dem oberen Ende des verbreiterten Randabschnitts 6 und der oberen Schräge 140d' der Aussparung ein ausreichendes axiales Spiel bereitgestellt sein. Insgesamt wird somit der verbreiterte Randabschnitt 6 von der Haltezunge 140 klammerartig und formschlüssig umgriffen. Die Einführschräge 140d', der gekrümmte Abschnitt 140b sowie die Schrägen 140d' der Aussparung ermöglichen dabei ein Einführen und Abziehen der Behälter ohne größeren Kraftaufwand in die Öffnungen bzw. aus diesen heraus unter elastischen Wegbiegen der Haltezungen 140.

Die Fig. 2h stellt eine stark vergrößerte Draufsicht auf eine Einführschräge einer Haltezunge gemäß einer Variante zu der Haltestruktur gemäß der Fig. 2a dar. Gemäß der Fig. 2h ist die Einführschräge 140d mittels eines darauf ausgebildeten bogenförmigen Grats 140f insgesamt verdrillt ausgebildet. Diese gewundene Einführschräge 140d ist auf sämtlichen Haltezungen der Öffnungen oder Aufnahmen gleich ausgebildet. Insgesamt sind die Einführschrägen, in Draufsicht betrachtet, um einen Winkel von kleiner 90° gekrümmt ausgebildet. Im Zusammenwirken mit dem Behälter bewirkt dies beim Einführen der Behälter in die Öffnungen, dass die Haltezungen nicht nur radial auswärts weggeschwenkt oder zurückgeklappt werden, sondern gleichzeitig mit einer Bewegungskomponente in Umfangsrichtung in Entsprechung zu der Geometrie der Einführschrägen 140d weggedreht werden, und zwar um einen Winkel von kleiner 90°. Je nach der Geometrie der Anordnung der Haltezungen auf dem Träger kann so eine Kollision von Haltezungen von unmittelbar benachbarten Öffnungen oder Aufnahmen beim Zurückschwenken oder Zurückklappen vermieden werden. Auf diese Weise kann die Packungsdichte der Behälter an der Haltestruktur noch weiter erhöht werden.

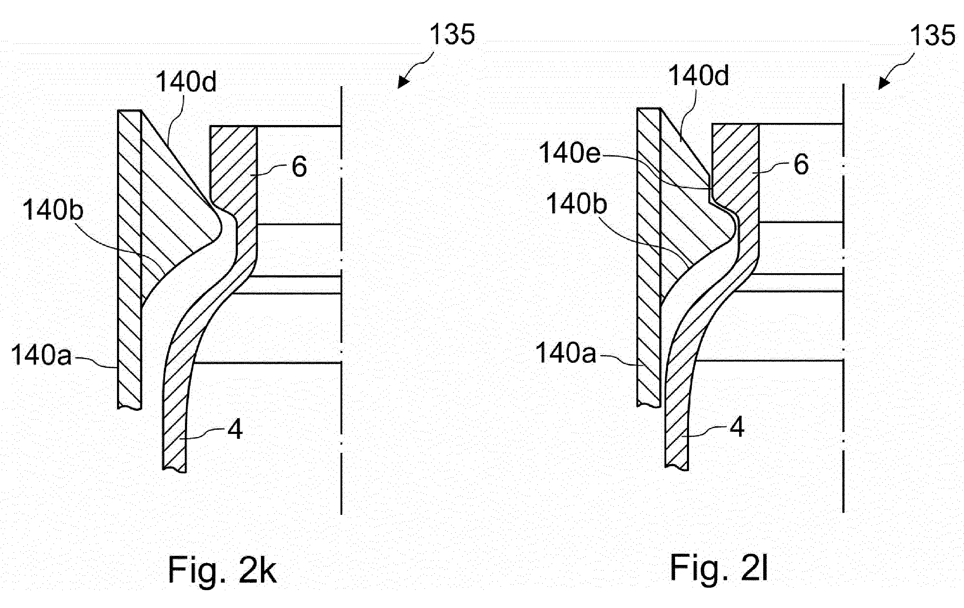

Die Fig. 2i zeigt in einer Draufsicht eine weitere Variante von Haltezungen für eine Haltestruktur gemäß der Fig. 2a, bei der die Basis 140a, in Axialrichtung betrachtet, verdrillt ausgebildet ist, was im Zusammenwirken der Einführschräge 140d mit dem Behälter beim Einführen des Behälters von oben her in die Öffnung oder Aufnahme sowohl eine radiale Komponente als auch eine Komponente in Umfangsrichtung beim elastischen Wegschwenken der Haltezungen ergibt, wie durch die beiden Doppelpfeile schematisch angedeutet. Die Fig. 2k zeigt schematisch die Halterung eines Behälters 2 in einer Haltestruktur, wie in der Fig. 2f gezeigt.

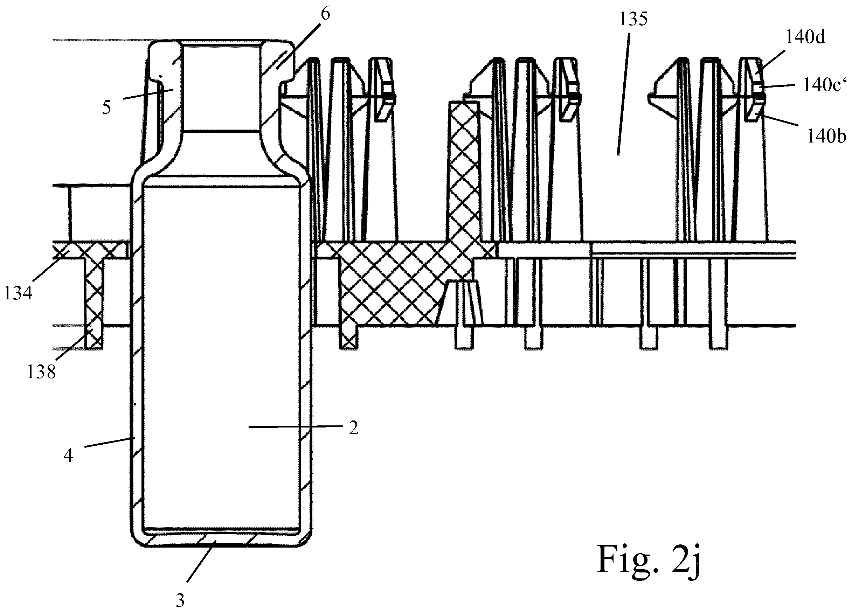

Die Fig. 2l zeigt eine weitere Variante zu der Ausführungsform nach der Fig. 2f mit modifizierter Ausbildung der fahnchenartigen elastischen Haltezungen 140. Während bei der Ausführungsform nach der Fig. 2f der Übergangsbereich zwischen den beiden Einführschrägen 140b und 140d abgeflacht oder auswärts abragend ausgebildet ist, ragt bei der Ausführungsform nach der Fig. 2l die untere Einführschräge 140b weiter in die Öffnung 135 hinein als die obere Einführschräge 140d. Der Übergangsbereich 140c' (vgl. Fig. 2j) erstreckt sich im Wesentlichen senkrecht oder verläuft relativ steil abwärts geneigt. Der obere Rollrand 6 des Fläschchens 2 kann lose auf diesem geneigten Übergangsbereich 140c' aufliegen oder auf einer Stufe, die von der Oberseite der unteren Einführschräge 140b ausgebildet wird. In jedem Fall sind die elastischen Haltezungen 140 so ausgelegt, dass zwischen den vorderen Enden der Haltezungen 140 und dem von diesen gehaltenen Fläschchen 2 ein gewisses radiales Spiel besteht, sodass insbesondere Fertigungstoleranzen der Fläschchen 2 ausgeglichen werden können.

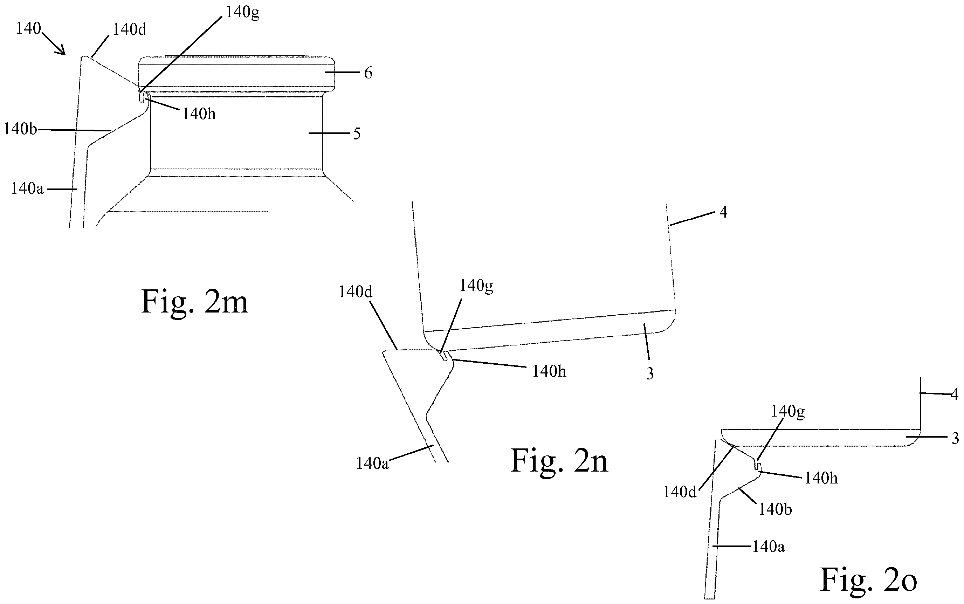

Die Figuren 2m bis 2o zeigen in einer Bewegungssequenz das Einführen eines Behälters in eine Haltestruktur gemäß einer weiteren Variante einer Haltestruktur. Gemäß der Fig. 2m ist an dem der zugeordneten Öffnung zugewandten vorderen Ende der oberen Einführschräge 140d eine Einkerbung 140g ausgebildet, der sich ein Vorsprung 140h anschließt, der eine vergleichsweise geringe Stärke aufweist und sich somit relativ leicht verbiegen lässt. In der Halteposition gemäß der Fig. 2m liegt die Unterseite des Rollrands 6 des Fläschchens lose auf diesem Vorsprung 140h auf. Dabei kann zwischen dem Rollrand 6 und dem der Öffnung zugewandten vorderen Ende der oberen Einführschräge ein radiales Spiel bestehen, wie vorstehend beschrieben. Die Fig. 2o zeigt, wie der Boden 3 des Fläschchens beim Einführen von oben her in die zugeordnete Öffnung entlang der oberen Einführschräge 140d hinabgleitet, bis schließlich gemäß der Fig. 2n der Vorsprung 140h erreicht ist. Sobald der Boden 3 mit seinem abgeschrägten Umfangsrand den Vorsprung 140h berührt, bewirkt die Kontaktkraft ein Eindrücken des Vorsprungs 140h, wodurch sich der Kontaktwinkel verändert (er wird steiler) und das weitere Abrutschen des Fläschchens über den vorderen Bereich der oberen Einführschräge 140d hinaus begünstigt wird. Beim Durchdrücken des Rollrands 6 übt dieser eine Kraft auf den Vorsprung 140h aus, sodass ein Moment auf die ganze Haltezunge 140 einwirkt, das die Haltezunge 140 nach innen biegen würde. Der Vorsprung 140h biegt sich jedoch in Richtung des Fläschchens und der Kontaktwinkel wird so verändert, dass die Kraft nun mehr in die Richtung des Fußes der Haltezunge wirkt, sodass das Moment, das die Haltezunge 140 nach innen biegt, vorteilhaft verringert werden kann.

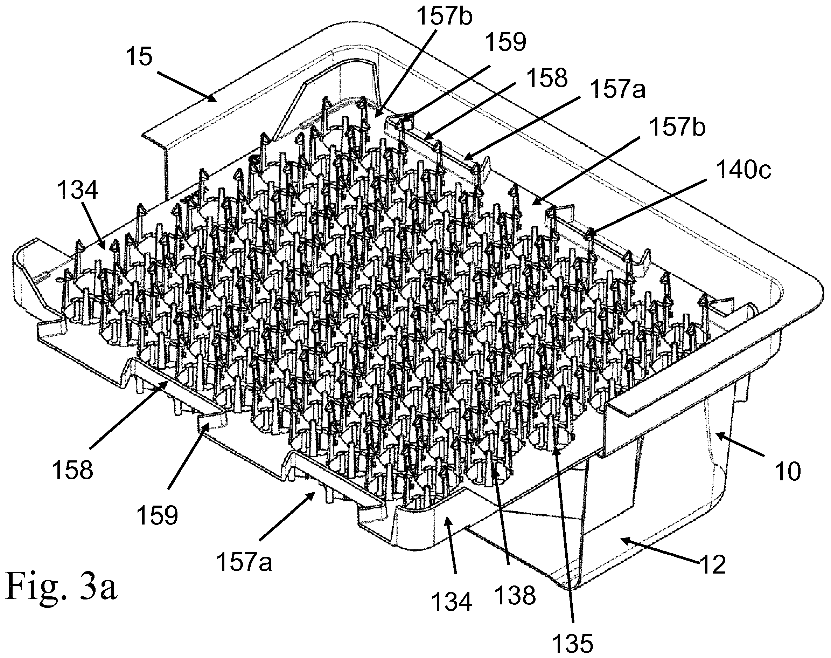

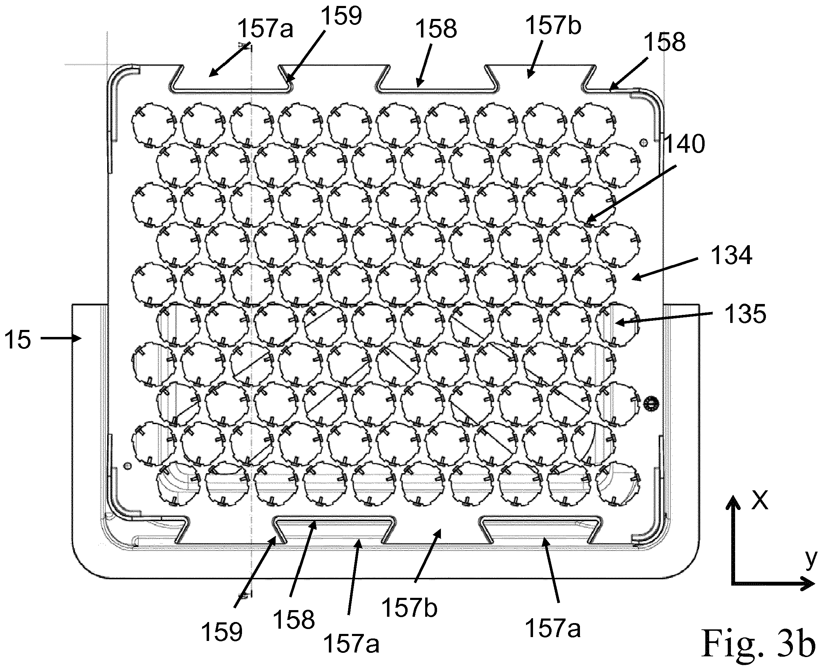

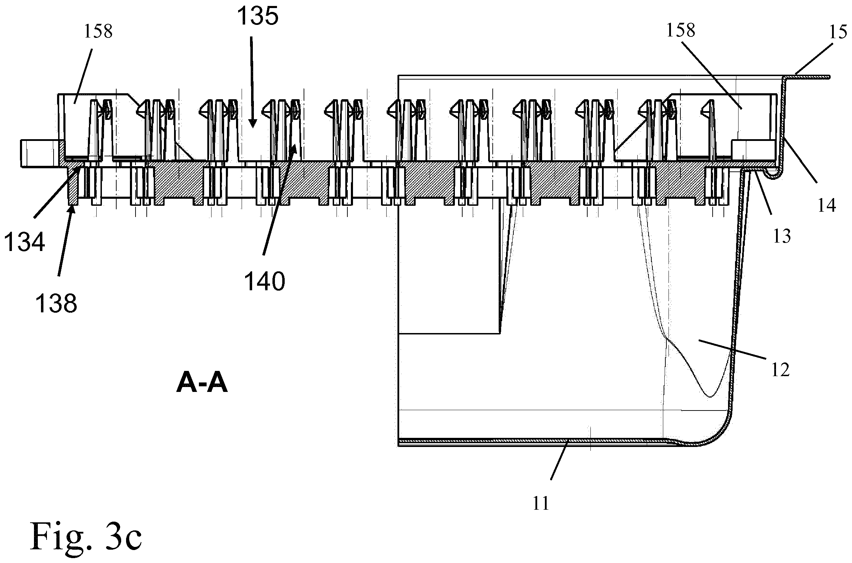

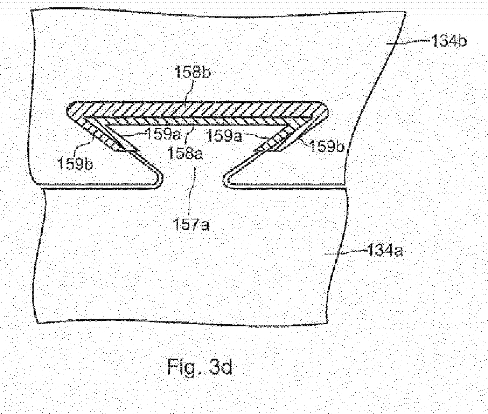

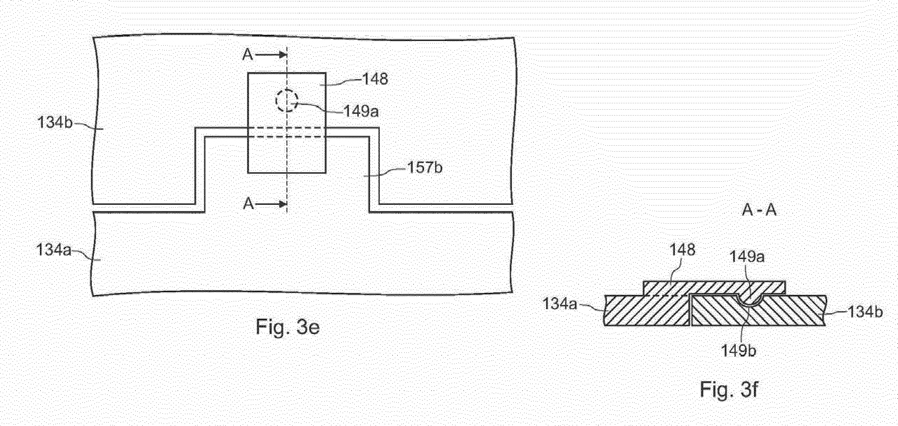

Die Fig. 3a zeigt eine Haltestruktur gemäß einer weiteren Ausführungsform in einer perspektivischen Draufsicht. Gemäß der Fig. 3a sind entlang der beiden Längsseiten der Halteplatte 134 alternierend und unter regelmäßigen Abständen zueinander eine Mehrzahl von Vorsprüngen 157b und Aussparungen 157a ausgebildet. Diese weisen, jeweils in Draufsicht betrachtet, eine insgesamt dreieckförmige oder polyedrische Grundfläche auf und sind korrespondierend zueinander ausgebildet, sodass diese unmittelbar miteinander verhakt werden können.

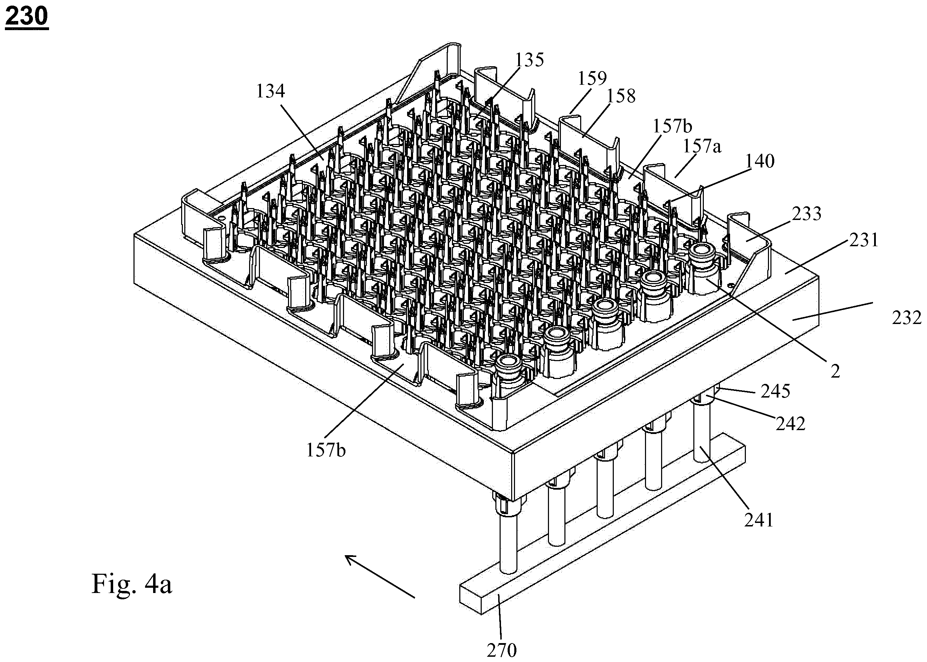

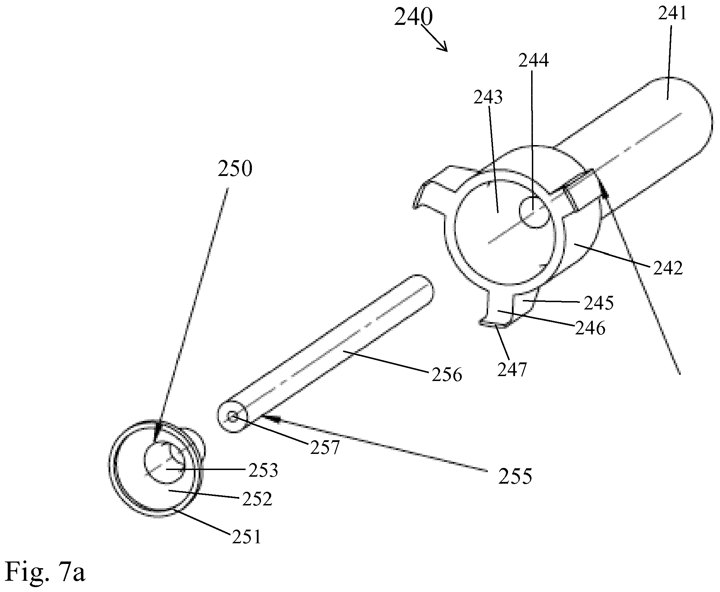

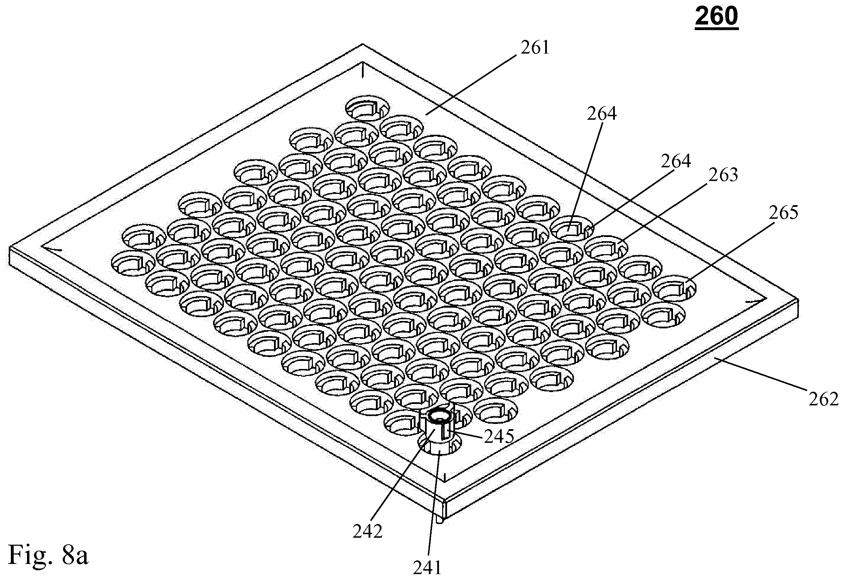

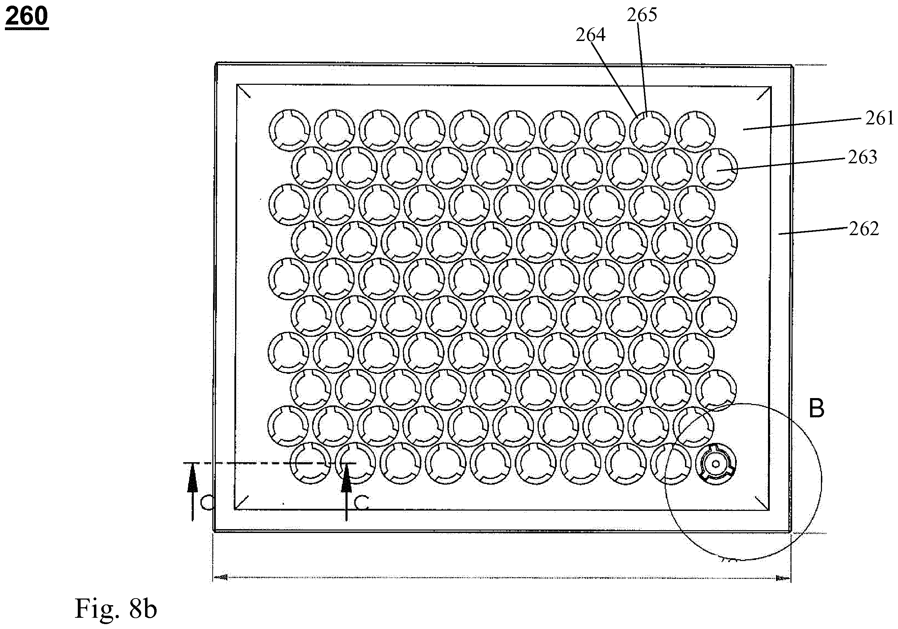

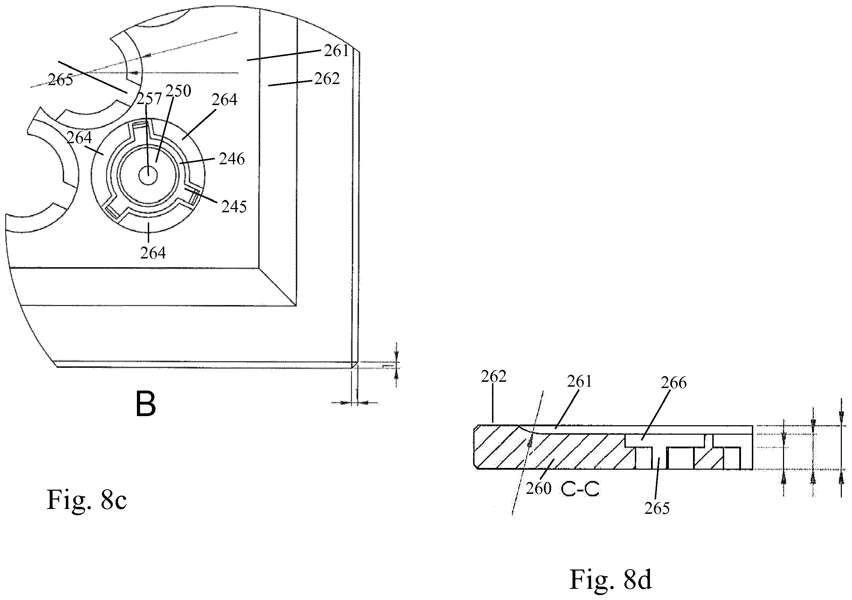

Die Fig. 4a zeigt einen Ausschnitt aus einer Bearbeitungsstation zur Bearbeitung oder Verarbeitung von Fläschchen, während diese in einer angehobenen Stellung in einer Haltestruktur gemäß der Fig. 2d gehalten sind. Die Bearbeitungsstation weist eine Verstelleinrichtung 230 mit einem Ausheber 270 auf, der mehrere Stößel (vgl. Fig. 7a) trägt und führt. Die Halteplatte 134 mit Ihren am Rand ausgebildeten Vorsprüngen 157b und Aussparungen 157a ist in der korrespondierend dazu ausgebildeten Führungsplatte 231 aufgenommen. In der von der Seitenwand 232 ausgebildeten Aufnahme kann eine Lochplatte, wie in der Fig. 8a dargestellt, exakt positioniert aufgenommen sein, um die Stößel exakt relativ zu der Halteplatte mit den daran gehaltenen Fläschchen 2 zu positionieren.

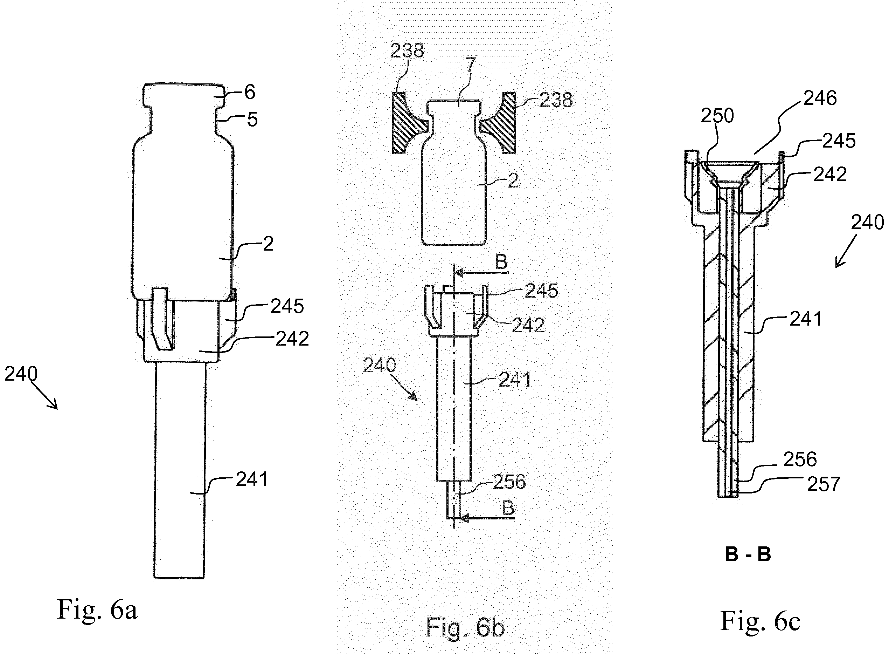

Gemäß der Fig. 7a weist jeder als Vertikal-Verstelleinrichtung wirkende Stößel 240 einen Schaft 241 mit einem Haltering 242 auf, von dessen Umfangsrand unter gleichmäßigen Winkelabständen zueinander drei Haltearme 245 abragen, deren Oberseiten gemeinsam eine Ebene aufspannen, die als Auflagefläche 246 dient, auf welcher der Boden des zu haltenden Fläschchens aufliegt, wie in der Fig. 6a dargestellt. Die Fläschchen werden von den Vorsprüngen 247, die senkrecht von den Haltearmen 245 abragen, gehalten. Dabei wird der untere Rand der Fläschchen von den Vorsprüngen 247 umgriffen, wodurch die Position der Fläschchen auf dem zugeordneten Stößel exakt vorgegeben werden kann und ein seitliches Verrutschen der Fläschchen, während diese von einem Stößel abgestützt sind, verhindert werden kann. Zu diesem Zweck können die Vorsprünge 247 den unteren Rand eines Fläschchens jeweils eng anliegend umgreifen, insbesondere klemmen. Zweckmäßig sind hierzu die Haltearme 246 und/oder Vorsprünge 247 ausreichend elastisch, beispielsweise aus einem Kunststoff ausgebildet, wodurch Toleranzen am unteren Ende der Fläschchen ohne weiteres kompensiert werden können.

Ein solcher Stößel kann ein Fläschchen gegen die von den Haltearmen der Halteplatte ausgeübte Haltekraft vertikal relativ zu der Halteplatte verstellen, um diese zur Bearbeitung oder Verarbeitung in einer Prozessstation in eine angehobene Position zu verschieben.

Wie in der Fig. 7a gezeigt, ist in dem Schaft 241 eine axiale Durchgangsbohrung 244 ausgebildet, die als axiale Führung für die rohrförmige Stange 256 eines Saugers 250 dient. In der Stange 256 ist eine axiale Durchgangsbohrung 257 ausgebildet, die mit einem Unterdruck-Erzeuger, beispielsweise einer Saugpumpe, verbunden ist. Die Stange 256 kann in dem Schaft 241 axial verstellt werden.

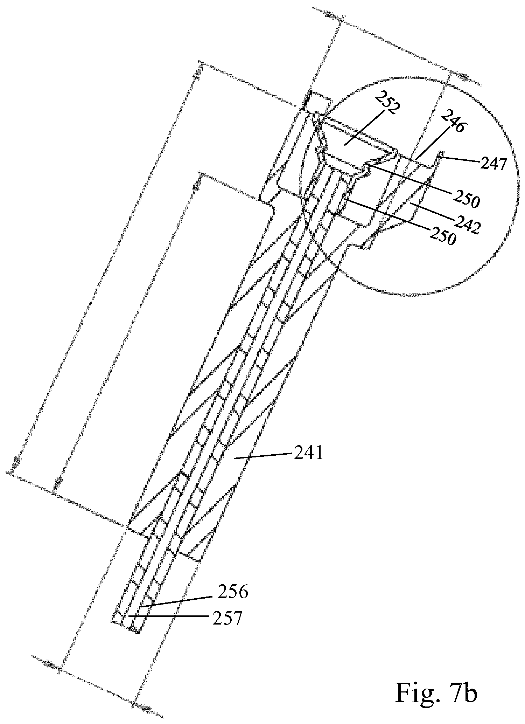

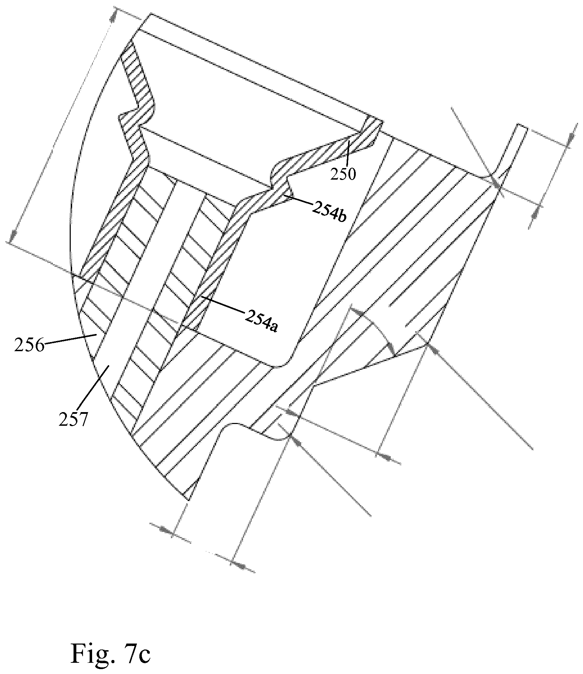

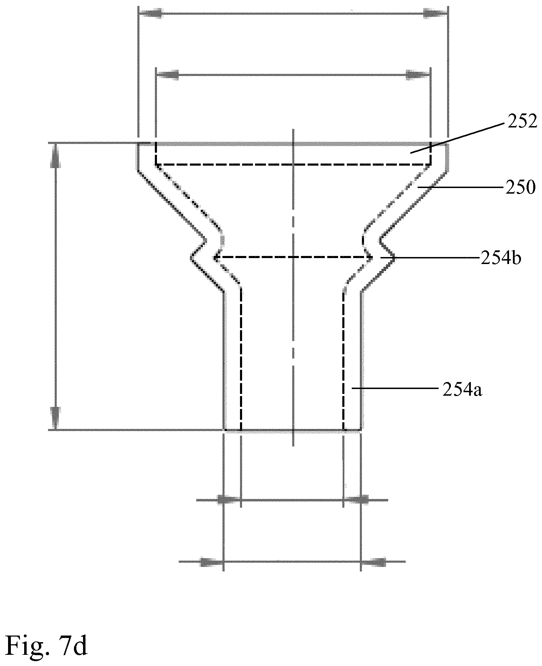

Auf das vordere freie Ende der Stange 256 ist eine Saugglocke 250 aus einem elastischen Material, beispielsweise Gummi oder Silikon, aufgesteckt. In der Saugglocke 250 ist ein trichterförmiger Hohlraum 252 ausgebildet, der über die Öffnung 253 und die Durchgangsbohrung 257 der Stange 256 mit dem Unterdruck-Erzeuger (nicht dargestellt) kommuniziert. Die Saugglocke 250 ist mit einer oder mehreren Wellungen 254b (vgl. Fig. 7c und 7d) versehen und kann sich durch Hochschieben der Stange 256 eng anliegend an den Boden des zugeordneten Fläschchens anschmiegen. Wie man der Fig. 7b entnehmen kann, kann die Saugglocke 250 vollständig in einer Aufnahme aufgenommen werden, die in dem Haltering 242 am vorderen Ende des Stößels ausgebildet ist. Die Saugglocke 250 kann in dieser Aufnahme vollständig versenkt werden, sodass diese nicht über die Auflagefläche 246 des Halterings 242 vorsteht. Allerdings kann die Saugglocke 250 durch Hochschieben der Stange 256 soweit vorgeschoben werden, dass diese schließlich in Kontakt mit dem Boden eines zu verstellenden Fläschchens gelangt. Dies erfordert eine gewisse Verschieblichkeit der Stange 256 relativ zu dem Schaft 241 des Stößels 240.

Nachfolgend wird anhand der Figuren 6a bis 6c ein Verfahren zur Bearbeitung oder Verarbeitung von Fläschchen zunächst grundlegend beschrieben.

Gemäß der Fig. 6a wird der Schaft 240 zum Anheben eines Fläschchens 2 in eine angehobene Position ausgehend von der Ruhestellung (vergleichbar zur Stellung gemäß der Fig. 6b) solange angehoben, bis der Haltering 242 am vorderen Ende des Schafts 241 in Anlage mit dem Boden eines Fläschchens 2 gelangt. In dieser Stellung ist der Boden des Fläschchens auf der Auflagefläche am vorderen Ende des Halterings 242 abgestützt und wird der untere Rand des Fläschchens von den Haltearmen 245 und den daran ausgebildeten Vorsprüngen 247 (vgl. Fig. 7a) umgriffen. In dieser Stellung kann die Saugglocke 250 in die Aufnahme 243 im Haltering 242 zurückgefahren sein.

In der angehobenen Position wird das Fläschchen entweder an weitere Fördermittel (nicht dargestellt) übergeben oder verbleibt in unmittelbarer Nähe des Stößels 240. Zur Bearbeitung oder Verarbeitung der Fläschchen können diese an oder in einer Prozessstation grundsätzlich mittels gesonderter Haltemittel 238, wie schematisch in der Fig. 6b gezeigt, gehalten werden. In einem solchen Fall kann der Stößel die Fläschchen 2 zur Bearbeitung oder Verarbeitung freigeben, beispielsweise nach unten oder seitlich weggefahren oder weggeschwenkt werden, wie in der Fig. 6b gezeigt.

Selbstverständlich kann jedoch der Stößel 240 auch alleinig zum Halten oder Abstützen der Fläschchen 2 während der Bearbeitung oder Verarbeitung verwendet werden. In einem solchem Fall verbleibt der Stößel dann zur Bearbeitung oder Verarbeitung an oder in der Prozessstation auf einer geeigneten Höhenposition und fixiert dabei die Position der Fläschchen 2 relativ zu der Prozessstation. Während der Bearbeitung oder Verarbeitung kann der Stößel dabei weiterhin zu einer geeigneten Verstellung der Fläschchen 2 in Axialrichtung und/oder zum Drehen der Fläschchen (der Stößel 240 wirkt gleichzeitig als Drehteller) und/oder Neigen oder Verschwenken der Fläschchen (der Stößel 240 wird verkippt) verwendet werden. Hierzu ist eine geeignete Haltekraft des Halterings 242 zum Halten der Fläschchen 2 vorzusehen.

Während der Bearbeitung oder Verarbeitung der Fläschchen 2 können diese dabei grundsätzlich auch weiterhin in den Öffnungen oder Aufnahmen der Haltestruktur aufgenommen oder zumindest geführt sein, beispielsweise um deren Position während der Bearbeitung oder Verarbeitung weiter zu stabilisieren. Grundsätzlich können die Fläschchen 2 während der Bearbeitung oder Verarbeitung jedoch auch vollständig aus den Öffnungen oder Aufnahmen der Haltestruktur herausgehoben und freigegeben sein.

Nach erfolgter Bearbeitung oder Verarbeitung in oder an einer Prozessstation sollen die Fläschchen wieder in eine tiefere Stellung zurückgezogen werden, insbesondere in die Öffnungen oder Aufnahmen einer Haltestruktur, wie vorstehend beschrieben. Hierzu wird, wie in dem Teilschnitt gemäß der Fig. 6c dargestellt, die Saugglocke 250 so positioniert, dass diese bündig zur Auflagefläche 246 des Halterings 242 ist oder geringfügig über diese hinausragt oder zu dieser zurückgefahren ist. Der Stößel 240 wird zum Absenken der Fläschchen 2 so positioniert, dass die Auflagefläche 246 erneut in Kontakt mit dem Boden des Fläschchens 2 gelangt. Sofern der Stößel 240 während Bearbeitung oder Verarbeitung der Fläschchen 2 nicht zurückgefahren wurde, ist hierzu keine erneute Verstellung erforderlich. Durch Anlegen eines Unterdrucks an die Saugglocke 250 schmiegt sich diese dem Boden des Fläschchens 2 an und kann dieser hin zu der Auflagefläche 246 des Stößels gesaugt werden. Ggf. kann nun das Haltemittel 238 der Prozessstation (vgl. Fig. 6b) das Fläschchen 2 wieder freigeben. Anschließend wird der Stößel 240 abgesenkt. Das Fläschchen folgt der Absenkbewegung des Stößels 240.

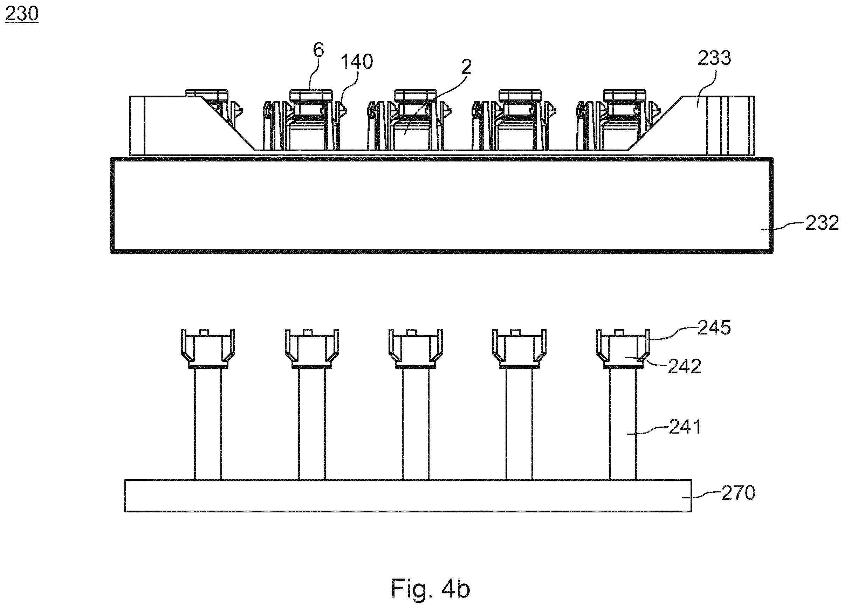

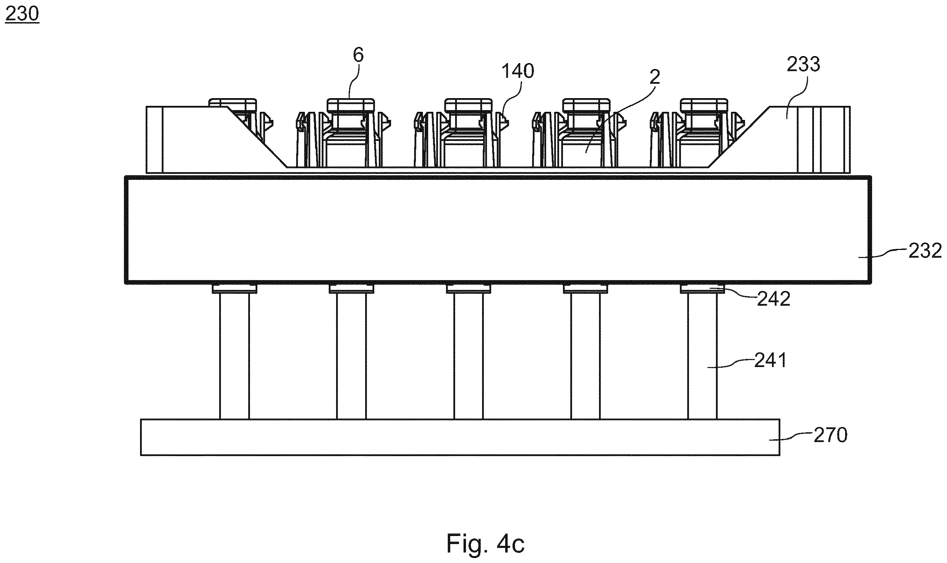

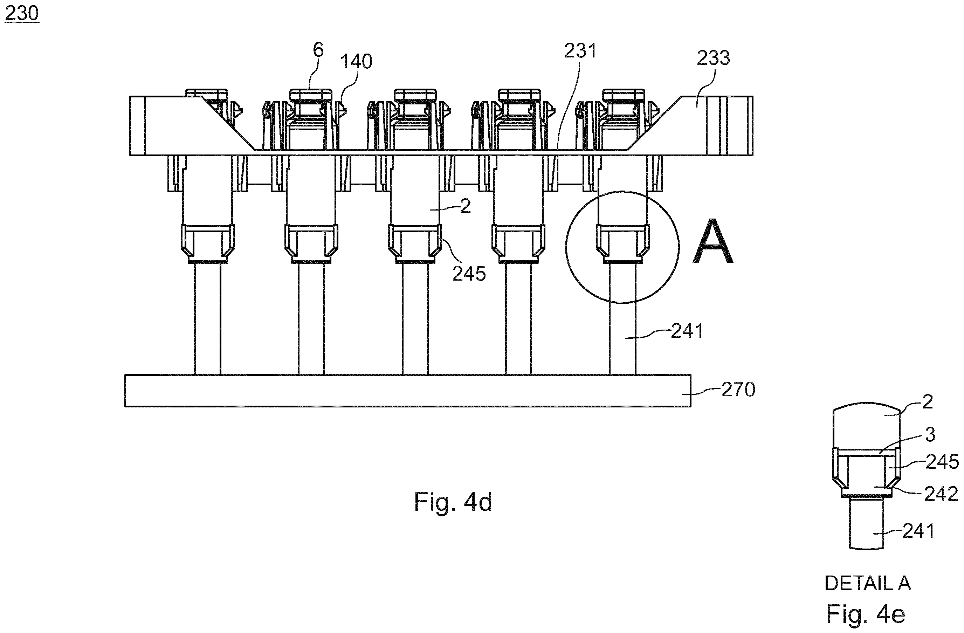

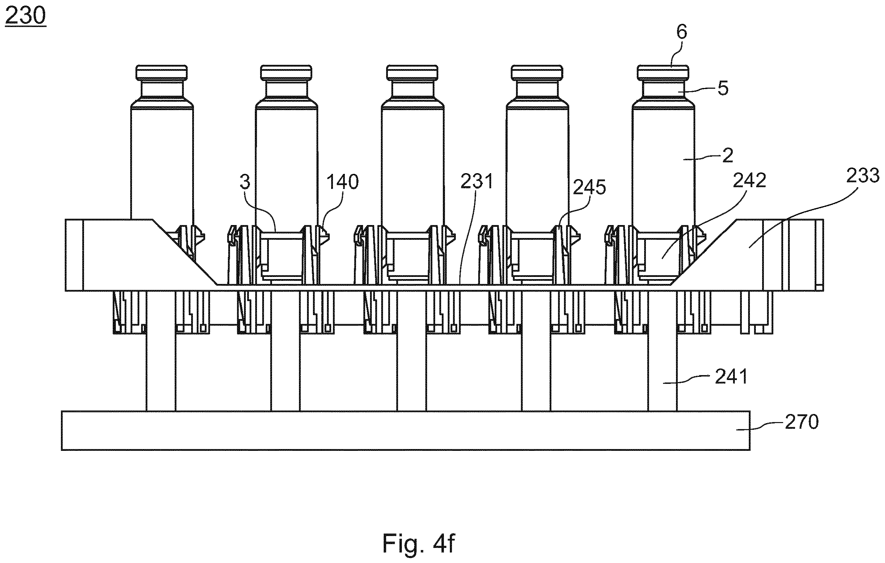

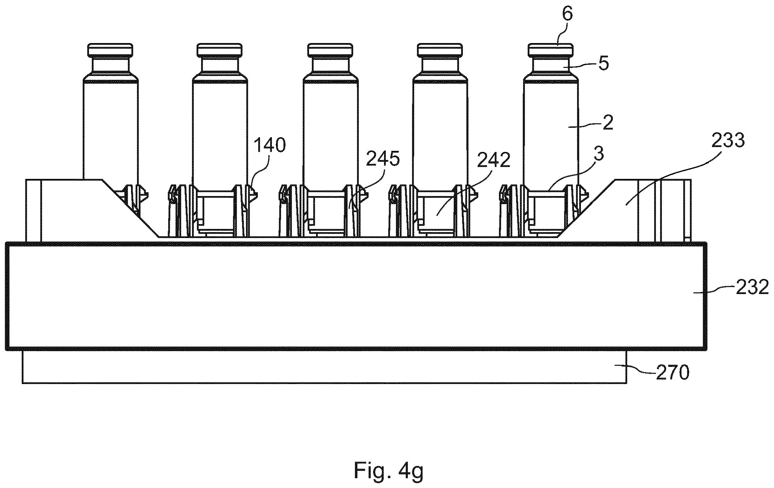

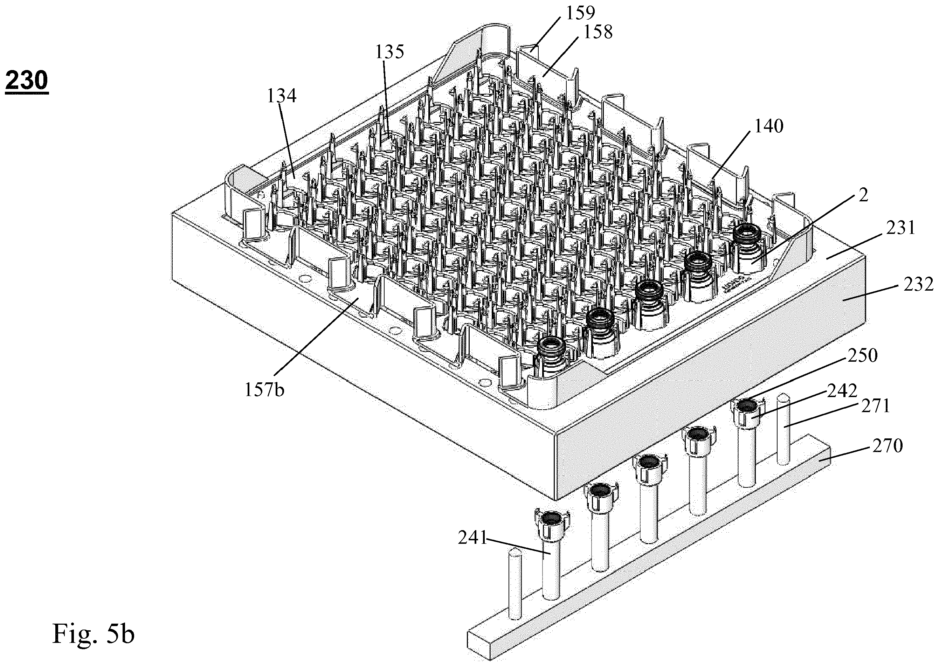

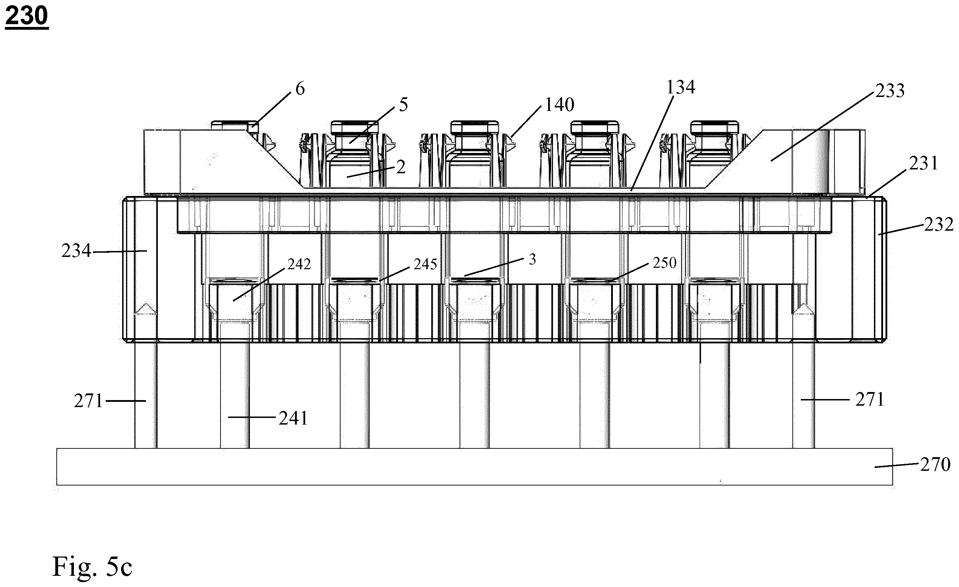

Die Figuren 4b bis 4g zeigen die vorstehend beschriebene Sequenz von Prozessschritten zum Überführen der Fläschchen in der Bearbeitungsstation gemäß der Fig. 4 aus einer abgesenkten Ausgangsstellung (Fig. 4b) in eine angehobene Stellung (Fig. 5h) und umgekehrt. Den Figuren 4b bis 4g liegt dabei eine Vertikal-Verstelleinrichtung 270 zugrunde, die eine Mehrzahl von Stößeln 241, wie vorstehend beschrieben, aufweist, die entsprechend der regelmäßigen Anordnung der Fläschchen 2 in der Haltestruktur angeordnet sind. Gemäß der Fig. 4b sind insgesamt fünf Stößel unter gleichmäßigen Abständen zueinander und parallel zu einer Längsseite der Haltestruktur verlaufend angeordnet, in Zuordnung zu den fünf Fläschchen 2, die in der Haltestruktur gehalten sind. Somit können durch gleichzeitiges Anhaben aller Stößel die von der Haltestruktur gehaltenen Fläschchen 2 reihenweise verstellt werden.

Zunächst werden die Fläschchen von einem Greifer gegriffen, wie beispielsweise in der Fig. 2j oder 2k dargestellt, oberhalb der Halteplatte gegriffen und dort gehalten. Oder die Fläschchen werden lose in die Öffnungen der Halteplatte eingesteckt oder eingelegt, wie in der Fig. 4b dargestellt. Die Stößel 240 fahren nun von unten vertikal hoch, wie in der den Figuren 4c und 4d (ohne Seitenwand 232) sowie in der stark vergrößerten Darstellung gemäß der Fig. 4e dargestellt. Dabei können die Haltearme 245 der Stößel 240 im Zusammenwirken mit den Aussparungen 265 einer Lochplatte 260 (vgl. Fig. 8a), wie nachfolgend beschrieben, für eine Zentrierung der Böden der Fläschchen 2 auf den Stößeln 240 sorgen. Anschließend werden die Stößel 240 weiter vertikal nach oben verstellt, um die Fläschchen 2 vertikal anzuheben. Ausgehend von der Stellung gemäß der Fig. 4c, in welcher die Fläschchen 2 von den elastischen Haltearmen 140 unterhalb des oberen Rands 6 gehalten werden, wie vorstehend beschrieben, werden beim Hochschieben der Fläschchen 2 die elastischen Haltearme 140 dabei aufgespreizt oder seitlich weggeschwenkt und gleiten dann entlang der Seitenwand der Fläschchen 2. Wie vorstehend beschrieben, können die Fläschchen 2 zur Bearbeitung oder Verarbeitung in einer Prozessstation vollständig über die Haltearme 140 hinaus angehoben werden, können jedoch auch grundsätzlich noch in einem Eingriff mit diesen stehen.

Das Anlegen eines Unterdrucks an die Saugglocke der Stößel bewirkt im Anschluss an die Bearbeitung oder Verarbeitung der Fläschchen 2 das Ansaugen der zugeordneten Fläschchen 2 an den Stößel 240. Durch Herunterziehen der Stößel 240 kann die von den Haltearmen 140 ausgeübte Halte- oder Rückstellkraft überwunden werden und die Fläschchen 2 so erneut in die abgesenkte Haltestellung in der Halteplatte überführt werden. Durch geeignete Ausgestaltung der Wellungen der Saugglocke (vgl. Fig. 7b) und geeignete Wahl deren Materials kann ein Abreißen des Kontakts zwischen Saugglocke und Fläschchenboden wirkungsvoll verhindert werden. Beim Absenken des Stößels wirkt dieser somit als elastischer Sauger. Der Greifer (vgl. Fig. 2j und 2k) kann nun entfernt werden; die Fläschchen können nach unten in die Haltestruktur (Nest) bewegt werden und wird dann dort erneut von den Haltemitteln an der Haltestruktur gehalten werden, wie vorstehend beschrieben.

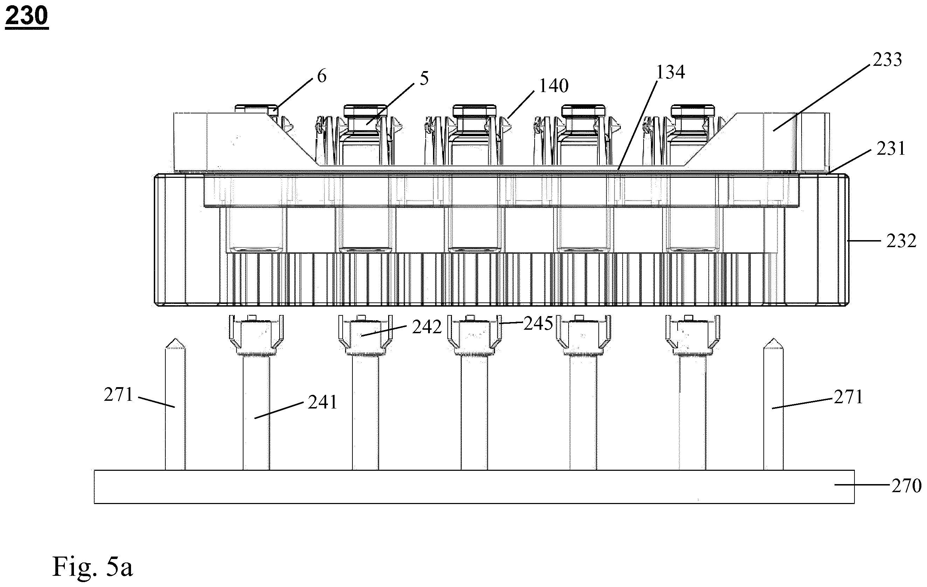

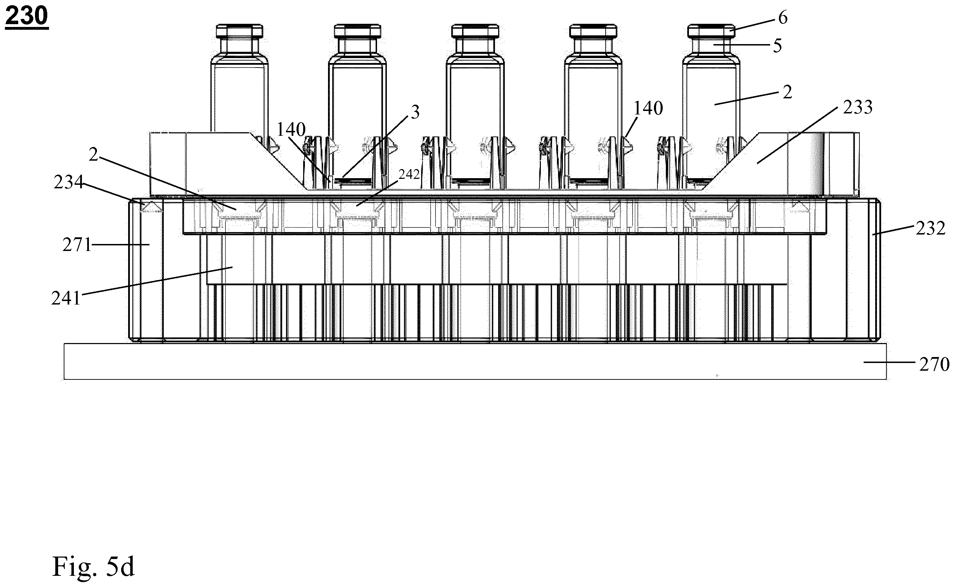

Die Figuren 5a bis 5e zeigen eine entsprechende Sequenz von Prozessschritten für eine weitere Ausführungsform, bei der an der Vertikal-Verstelleinrichtung 270 ferner mehrere zylindrische Positionierungszapfen 271 vorgesehen sind, die im Zusammenwirken mit einer korrespondierend ausgebildeten Positionierungshülse 234 in der Seitenwand 232 der Prozessstation 230 oder der Haltestruktur 134 für eine präzise Relativ-Positionierung von Vertikal-Verstelleinrichtung 270 und Haltestruktur 134 sorgen. In der angehobenen Stellung der Stößel 240 gemäß der Fig. 5d gelangt das vordere Ende der Positionierungszapfen 271 in Anlage zu dem Träger 134, wodurch gleichzeitig auch ein weiteres Anheben der Stößel 240 unterbunden werden kann.

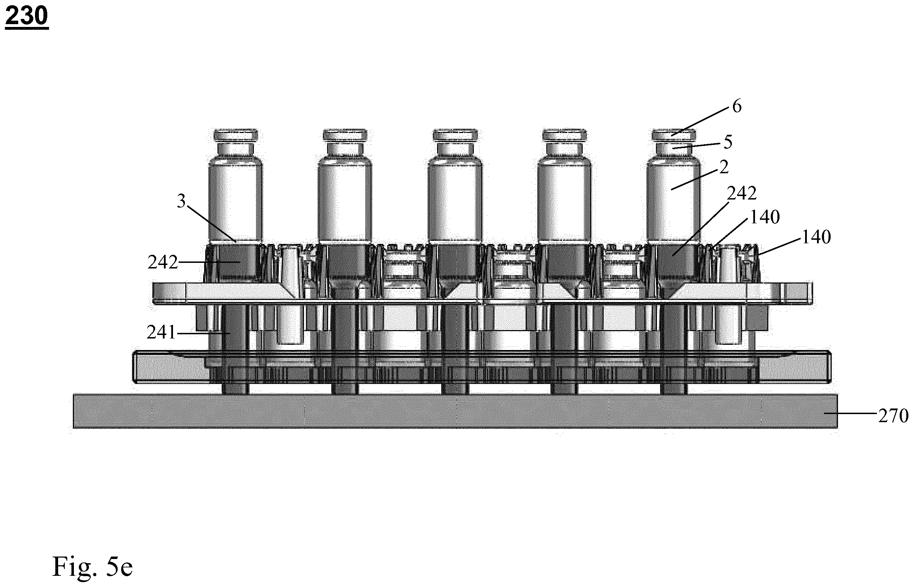

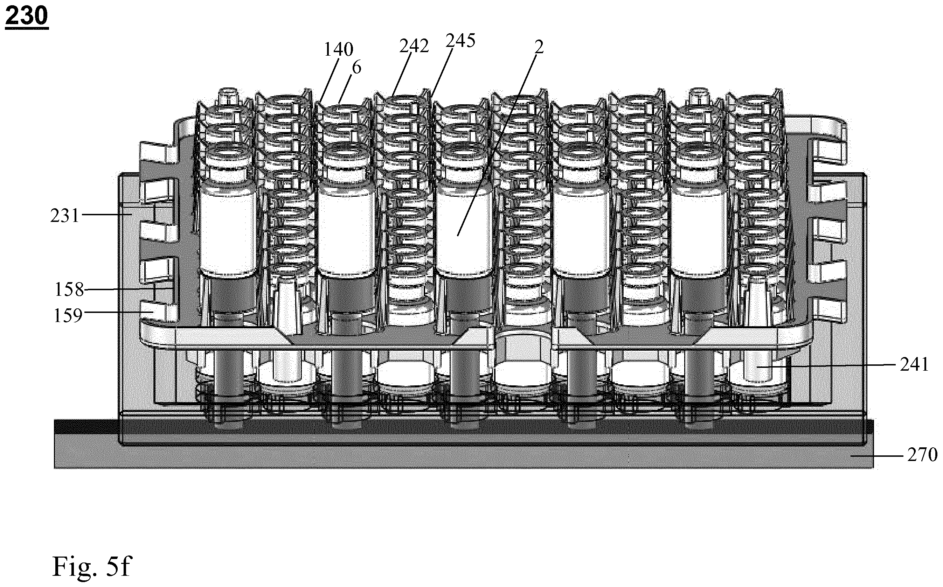

Die Fig. 5e zeigt dabei die Prozessstation 230 in der Stellung gemäß der Fig. 5d, jedoch ohne den Blick auf die Fläschchen und Stößel verdeckende Seitenwände. Schließlich zeigt die Fig. 5f die Prozessstation in einer Stellung der Vertikal-Verstelleinrichtung 270, in welcher der Eingriff sämtlicher Behälter 2 mit den Haltearmen 140 der Haltestruktur vollständig gelöst ist. Dabei ist in der Fig. 5f jede zweite Reihe von Fläschchen 2 nicht dargestellt, um den Blick auf die oberen Enden der Stößel 241 mit dem Haltering 242 und den Haltearmen 245 in der angehobenen Stellung der Stößel 241 freizugeben.

Zum Ausheben der Fläschchen braucht kein Unterdruck an die Saugglocken der Stößel angelegt zu werden. Bei ganz ausgefahrener Position (oberste Stellung) der Stößel kann allerdings durch Unterdruck eine zusätzliche Sicherung der Fläschchen ermöglicht werden.

Zur exakten Positionierung der Stößel 240 in Übereinstimmung mit der Anordnung der Fläschchen in der Halteplatte kann eine in der Fig. 8a dargestellte Lochplatte 260 vorgesehen sein. In der Lochplatte 260 ist für jedes Fläschchen ein Loch 263 mit ringförmiger Auflage 266 vorgesehen, die durch eine Mehrzahl von unter gleichen Winkelabständen zueinander in dem Loch 263 angeordneten gekrümmten Halteteilen 264 ausgebildet wird, die von Aussparungen 265 unterbrochen sind, durch welche die jeweiligen Haltearme 245 der Stößel 240 nach oben geführt werden können. Diese Lochplatte 260 kann geeignet unterhalb der Haltestruktur mit den von dieser gehaltenen Fläschchen angeordnet sein und die Stößel und deren Haltearme beim Anheben so führen, dass die Haltearme den unteren Rand der Fläschchen präzise umgreifen.

Gemäß einer weiteren Ausführungsform kann die in der Fig. 8a abgebildete Lochplatte 260 auch als Haltestruktur zum Halten der Fläschchen (nicht gezeigt) verwendet werden, in welchem Fall die Böden der Fläschchen in den kreisförmigen Aussparungen 265 aufgenommen sind und unmittelbar auf den gekrümmten Halteteilen 264 aufliegen, um an der Lochplatte abgestützt zu sein.

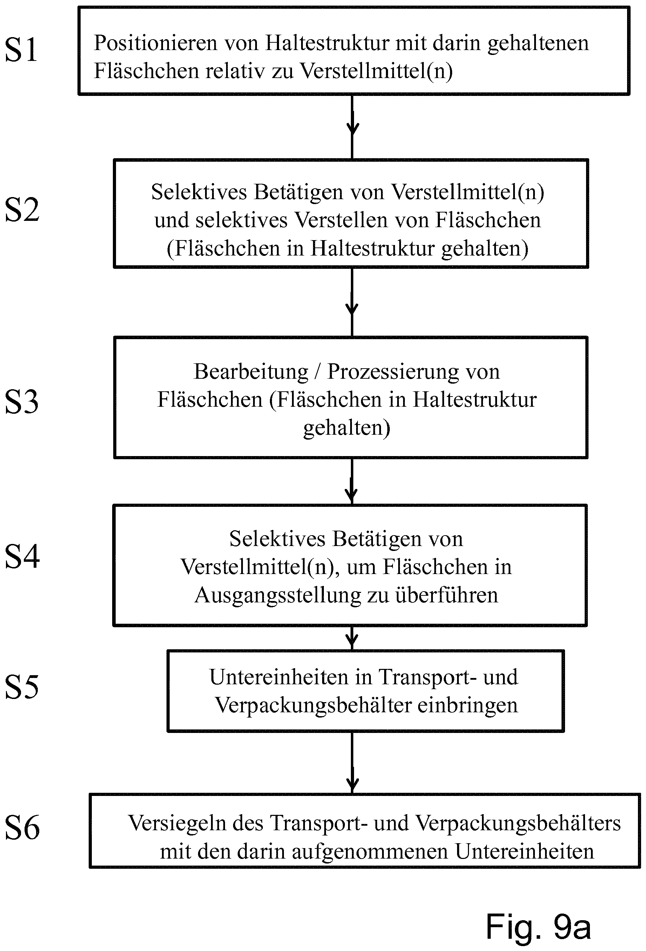

Das schematische Flussdiagramm gemäß der Fig. 9a fasst die Schritte zur Prozessierung oder Bearbeitung von Fläschchen mittels einer Bearbeitungsstation gemäß der Fig. 4a nochmals zusammen.

Somit wird die jeweilige Haltestruktur mit den daran gehaltenen Behältern, insbesondere Fläschchen (vials), Ampullen, Karpulen oder Spritzenkörpern, in dem Prozessschritt S1 relativ zu der Vertikal-Verstelleinrichtung geeignet positioniert. Diese Positionierung kann bereits dann erfolgen, wenn die Haltestruktur noch in dem Transport- und Verpackungsbehälter aufgenommen ist, wie beispielhaft in der Fig. 2a dargestellt. In einem solchen Fall könnte die Haltestruktur 134 beispielsweise mittels eines Greifers oder dergleichen über die Zugriffsöffnungen 29 (vgl. Draufsicht gemäß der Fig. 2b) gegriffen und fixiert und dann der Transport- und Verpackungsbehälter 1 nach unten abgezogen werden. Anschließend kann die Vertikal-Verstelleinrichtung, beispielsweise wie in der Fig. 4a dargestellt, die Behälter reihenweise in die angehobene Position anheben (Prozessschritt S2), in welcher die Behälter dann bearbeitet oder weiterverarbeitet werden (Prozessschritt S3).

Bei dieser Bearbeitung oder Verarbeitung kann es sich um beliebige Prozessschritte handeln. Beispielhaft seien aufgeführt: ein Füllen der Behälter mit einer Substanz oder substanzhaltigen Lösung; eine Sterilisierung oder Wärmebehandlung der Behälter; ein Waschvorgang; eine Wärmbehandlung oder anderweitige Behandlung des Inhalts der Behälter, beispielsweise Bestrahlung; ein Verschließen der Behälter, beispielsweise mit einem Stopfen; das Aufbringen eines Metalldeckels auf einem Verschluss am oberen Rand der Behälter, beispielsweise durch Bördeln oder Crimpen einer Metallkappe; ein Beschriften oder Labeln der Behälter; ein Wiegen der Behälter. Diese Bearbeitung oder Verarbeitung der Behälter in der angehobenen Position im Prozessschritt S3 kann erfolgen, wenn die Behälter vollständig von den Haltemitteln der Haltestruktur freigegeben sind, kann jedoch grundsätzlich auch erfolgen, während die Behälter auch weiterhin in den Öffnungen oder Aufnahmen der Haltestruktur aufgenommen oder zumindest geführt oder in ihrer Position geführt oder fixiert sind.

Anschließend wird eine Vertikal-Verstelleinrichtung, die identisch zu der zuvor verwendeten Vertikal-Verstelleinrichtung sein kann aber auch eine andere Vertikal-Verstelleinrichtung sein kann, in dem Prozessschritt S4 betätigt, um die Behälter in ihre abgesenkte Ausgangsstellung zu überführen, also insbesondere durch Herunterziehen in die Öffnungen oder Aufnahmen der Haltestruktur, wie vorstehend beschrieben. In der abgesenkten Ausgangsstellung können die Behälter erneut in die Haltemittel der Haltestruktur eingreifen, um von diesen erneut gehalten oder abgestützt zu sein, was jedoch nicht zwingend erforderlich ist.

Anschließend kann die Haltestruktur mit den daran gehaltenen Behältern optional in den zuvor verwendeten Transport- und Verpackungsbehälter oder einen anderen Transport- und Verpackungsbehälter eingebracht werden und dieser für einen sterilen Transport in der üblichen Weise verschlossen oder versiegelt werden (Prozessschritt S6), beispielsweise durch Aufkleben einer sterilen Schutzfolie, beispielsweise einer Tyvek®-Folie.

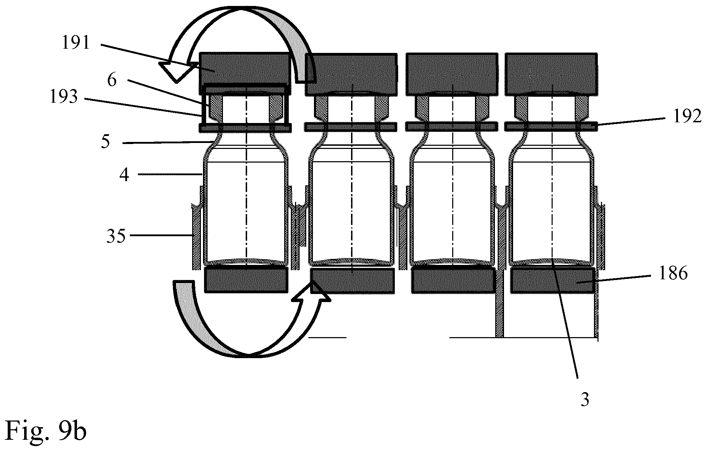

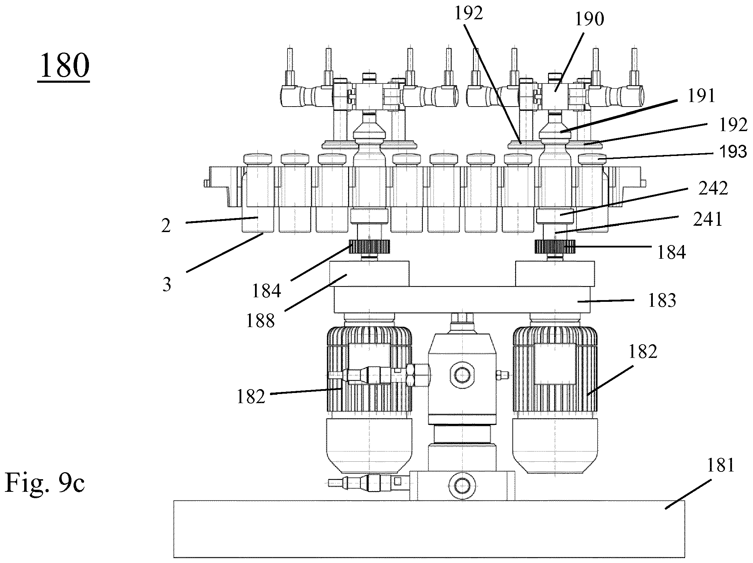

Die Fig. 9b zeigt als Beispiel für eine Bearbeitung der Behälter in der angehobenen Stellung das Aufbringen eines Metalldeckels auf den oberen Rand der Behälter. Wie man der vergrößerten Darstellung gemäß der Fig. 9b entnehmen kann, ist in der angehobenen Stellung der obere Rand der Behälter 2 mit dem darin eingesetzten Stopfen und einem Metalldeckel 193, beispielsweise einer Aluminiumkappe, in einer zugeordneten Zentrierscheibe 191 aufgenommen, um die Drehbewegung des Behälters beim Drehen des Drehtellers 186 zu zentrieren. Dieser Drehteller 186 kann Bestandteil der vorgenannten Vertikal-Verstelleinrichtung sein oder erst nach Anheben der Behälter in die angehobene Stellung mit diesen in Eingriff gebracht werden. Beim Drehen des Behälters 2 gelangt eine an dem Arm 190 gelagerte Bördelscheibe 192 in Anlage mit dem Metalldeckel 193 und bördelt diesen durch Umformung geeignet um, um so den Behälter steril zu versiegeln. Wenn sämtliche Behälter 2 eines Trägers in der vorstehend beschriebenen Weise verarbeitet worden sind, wird der Träger aus dem Bereich der Bördelstation 180 (vgl. Fig. 9c) abtransportiert und in der Prozessanlage (nicht dargestellt) weiter gefördert. Hierzu können die Behälter in den Öffnungen oder Aufnahmen des Trägers erneut in deren Normalstellung zurückgezogen und die Träger erneut in die Stellung gemäß der Fig. 2b überführt werden, beispielsweise durch Einsetzen in einen Transport- und Verpackungsbehälter, weiter gefördert zu werden. Die Verarbeitung in der Bördelstation erfolgt somit chargenweise, ohne dass die Behälter jedenfalls vollständig aus dem Träger entnommen zu werden brauchen.

Bei der Kappe, die gemäß der Fig. 9b auf den oberen Rand der Fläschchen aufgesetzt ist, kann es sich auch um sog. pre-fit caps handeln, wie nachfolgend beschrieben.

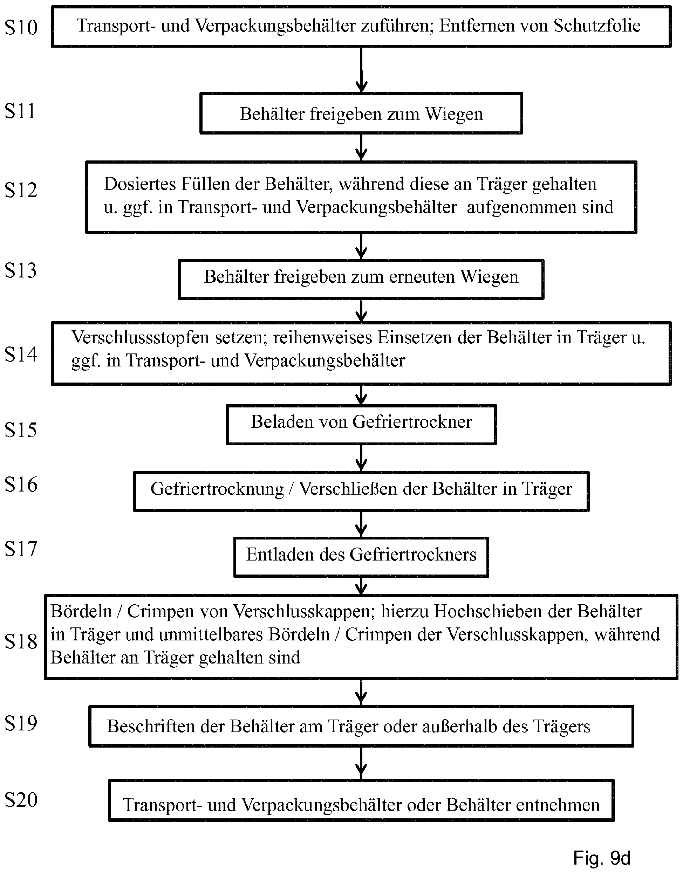

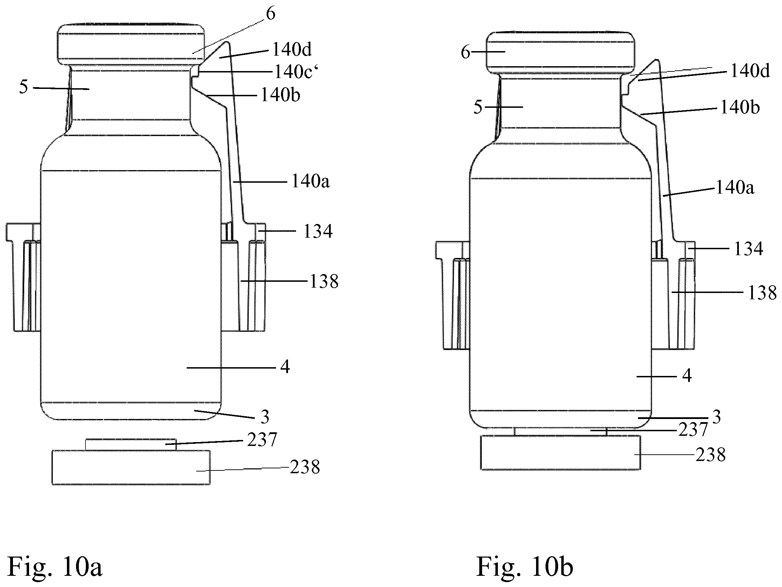

Die Fig. 9d zeigt als weiteres Beispiel ein schematisches Flussdiagramm eines Verfahrens gemäß einem weiteren Ausführungsbeispiel nach der vorliegenden Erfindung. Bei diesem Verfahren werden die Behälter vor dem Befüllen (Prozessschritt S11) und nach dem Befüllen (Prozessschritt S13) gewogen, sodass man eine genaue Information über die eingefüllte Menge erhält, die den einzelnen Behältern beispielsweise mittels einer Software oder durch Beschriftung oder Markierung rückverfolgbar zuordnen kann. Zum Wiegen der Behälter kann eine Wiegezelle verwendet werden, wie diese beispielhaft in den Figuren 10a und 10b dargestellt ist.

Die Wiegezelle umfasst eine Abstützfläche 238 und einen darauf vorgesehenen Gewichtssensor 237. Die Wiegezelle wird in den Prozessschritten S11 und S13 unter den Behältern positioniert, sodass der Gewichtssensor 237 das Gewicht des jeweils zugeordneten Behälters messen kann. Grundsätzlich kann diese Abstützfläche 238 identisch zu der Ablagefläche 246 am oberen Ende der Vertikal-Verstelleinrichtung sein (vgl. Fig. 7a) und der Gewichtssensor 237 ebenfalls in die Ablagefläche 246 der Vertikal-Verstelleinrichtung integriert sein, d.h. die Wiegezelle kann in die Vertikal-Verstelleinrichtung integriert sein. Alternativ kann die Wiegezelle auch als separate Messeinheit zu einem geeigneten Zeitpunkt unter die Behälter gefahren werden, um diese zu vermessen.

Wie in der Fig. 10b gezeigt, kann das Wiegen der Behälter grundsätzlich erfolgen, während die Behälter in den Öffnungen oder Aufnahmen der Haltestruktur aufgenommen sind, nämlich dann, wenn diese mit radialem und axialem Spiel oder mit radialem Spiel und axial frei verschiebbar von den Haltearmen 140a gehalten werden, wie vorstehend anhand der Figuren 2a bis 2o beschrieben, und die Behälter somit nur lose auf den Haltearmen 140 aufliegen, jedoch nicht in irgendeiner Weise von den Haltearmen geklemmt werden, was die Ergebnisse der Gewichtsmessung verfälschen würde. Natürlich kann das Wiegen in den Prozessschritten S11 und S13 auch erfolgen, wenn die Behälter vollständig aus den Öffnungen oder Aufnahmen der Haltestruktur herausgenommen sind.

Den Figuren 2c und 2j kann man entnehmen, dass bei den meisten Ausführungsformen einer Haltestruktur im Sinne der vorliegenden Erfindung die Böden der Behälter von der Unterseite der Haltestruktur ungehindert und vollflächig zugänglich sind. Dies ermöglicht einen unmittelbaren Kontakt der Böden der Behälter mit einer Kühlfläche oder einem Kühlfinger eines Gefriertrockners, um in dem Prozessschritt S16 an den Behältern eine Gefriertrocknung vorzunehmen. Hierzu ist, anders als nach dem Stand der Technik, keine aufwändige Entnahme der Behälter aus der Haltestruktur und Vereinzelung der Behälter erforderlich. Vielmehr kann die Gefriertrocknung ausgeführt werden, während die Behälter an der Haltestruktur gehalten sind.

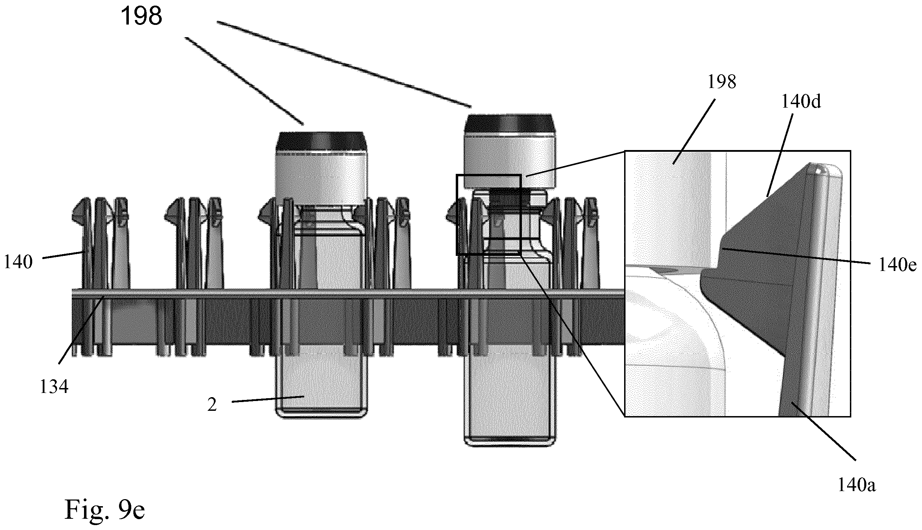

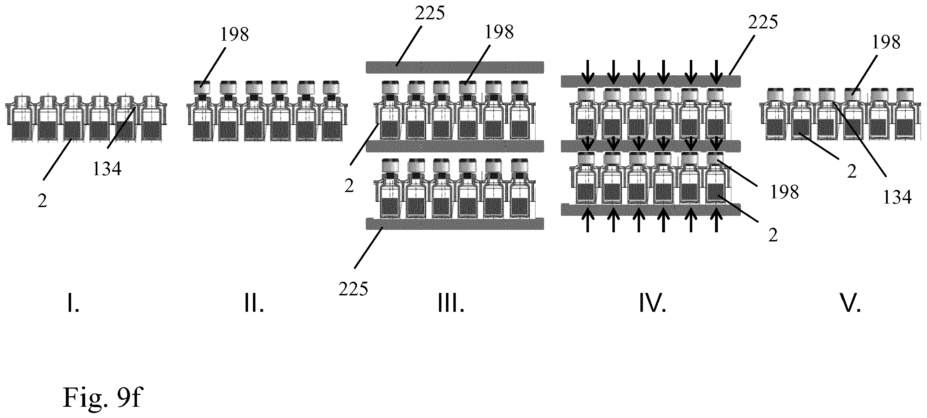

Die Figuren 9e und 9f zeigen ein Verfahren gemäß der vorliegenden Erfindung zum Verschließen von Fläschchen mit Hilfe von steril verschließenden Kappen, die unmittelbar auf den oberen Rand der Fläschchen aufgedrückt werden. Während die Fläschchen 2 in einer angehobenen Position der Kappe 198, also wie für das rechte Fläschchen 2 in der Fig. 9e dargestellt, über die bereits auf den oberen Rand des Fläschchens 2 aufgesetzte Kappe 198 befüllt werden kann oder zumindest weiter bearbeitet oder behandelt werden können, beispielsweise gefriergetrocknet werden können, weil das Innenvolumen der Fläschchen 2 mit der Umgebung kommuniziert, sind die Fläschchen 2 nach Herunterdrücken der Kappen 198, also wie für das linke Fläschchen 2 in der Fig. 9e dargestellt, steril verschlossen, ohne dass hierzu ein weiteres Bördeln oder Crimpen eines Metalldeckels erforderlich wäre. Zu diesem Zweck eignen sich insbesondere sog. pre-fit caps. Die vergrößerte Teildarstellung im rechten Bildteil der Fig. 9e zeigt die Halterung des Fläschchens 2 an den elastischen Haltezungen 140a des Trägers. Wie hier dargestellt, ist der untere Rand der Kappe 198 in der angehobenen Position am unteren Rand der Halteaufnahme 140e mit radialem Spiel abgestützt, liegt also locker auf der elastischen Haltezunge 140a auf. In dieser Position kann das Gewicht des Fläschchens 2 gemessen werden, beispielsweise mittels einer Wiegezelle, wie anhand der Figuren 10a und 10b beschrieben.

Wie man der Darstellung für die Halterung des linken Fläschchens 2 in der Fig. 9e entnehmen kann, ist der untere Rand der Kappe 198 in der herabgedrückten Position der Kappe 198 auch weiterhin am unteren Rand der Halteaufnahme 140e mit radialem Spiel abgestützt. Auch in dieser Position kann das Gewicht des Fläschchens 2 gemessen werden, beispielsweise mittels einer Wiegezelle, wie anhand der Figuren 10a und 10b beschrieben.

Zum sterilen Verschließen der Fläschchen 2 ist es somit wichtig, dass Kappe 198 und Fläschchen 2 relativ zueinander axial verstellt werden können. Diese axiale Verstellung kann erfindungsgemäß auch erfolgen, während die Fläschchen 2 an einem Träger (Englisch "nest") gehalten oder zumindest in dessen Öffnungen oder Aufnahmen geführt aufgenommen sind. Zusätzlich kann erfindungsgemäß auch ein Wiegen der Fläschchen 2 erfolgen, während diese an dem Träger gehalten sind.

Die Fig. 9f fasst fünf aufeinanderfolgende Prozessschritte (I. bis V.) zum Verschließen der Fläschchen gemäß einer weiteren Variante des Verfahrens nach der Fig. 9d zusammen. Zunächst werden die Fläschchen 2 in dem Prozessschritt I. befüllt, während diese an dem Träger 134 gehalten sind. Nach dem Befüllen werden die Kappen 198 in dem Prozessschritt II. auf den oberen Rand der Fläschchen 2 aufgedrückt. In dieser angehobenen Position der Kappen 198 kann eine Gefriertrocknung des Inhalts der Fläschchen 2 erfolgen, oder auch eine beliebige andere Behandlung oder Weiterverarbeitung der Fläschchen 2. Beispielhaft ist dies in dem Prozessschritt III. durch die Bezugszeichen 225 angedeutet, die Kühlböden eines nicht näher dargestellten Gefriertrockenschranks symbolisieren sollen. In dem Prozessschritt IV. werden die Kühlböden 225, oder andere Ablageflächen, auf denen die Böden der Fläschchen 2 unmittelbar aufliegen, gegeneinandergedrückt, wie durch die Pfeile angedeutet. Dadurch werden die Kappen 198 auf die Fläschchen 2 herabgedrückt und diese somit steril verschlossen. Die vorgenannten Prozessschritte können ausgeführt werden, während die Fläschchen 2 an einem Träger (Englisch "nest") gehalten oder zumindest in dessen Öffnungen oder Aufnahmen geführt aufgenommen sind. Anschließend folgt im Prozessschritt V. die Entnahme der Träger 134 mit den daran gehaltenen Fläschchen 2 aus der Prozessstation.

Wie dem Fachmann beim Studium der vorstehenden Beschreibung ohne weiteres ersichtlich sein wird, können die vorstehend beispielhaft beschriebenen Haltestrukturen und Vorgehensweisen zum Verstellen der Vertikal-Verstelleinrichtung und der Haltemittel auch in anderer Weise miteinander kombiniert werden, als vorstehend beschrieben.

Wie dem Fachmann beim Studium der vorstehenden Beschreibung ohne weiteres ersichtlich sein wird, ist der vorgenannte Gesichtspunkt der form- oder kraftschlüssigen Verbindung von unmittelbar benachbarten Haltestrukturen grundsätzlich unabhängig von der konkreten Ausführung der Halterung der Fläschchen an solchen Haltestrukturen, sodass dieser Gesichtspunkt grundsätzlich auch als unabhängiger Gesichtspunkt der vorliegenden Erfindung unabhängig von der konkreten Realisierung der Halterung der Fläschchen an solchen Haltestrukturen beansprucht werden kann.

Die von den Haltemitteln jeweils auf die Behälter ausgeübte Haltekraft ist ausreichend, um die Behälter zuverlässig an der Haltestruktur zu halten. Insbesondere ist die ausgeübte Haltekraft größer als die Gewichtskraft der Behälter, ggf. mit Inhalt und Verschlussstopfen. Dadurch wird eine zuverlässige Halterung der Behälter an der Haltestruktur gewährleistet. Gleichzeitig können die Behälter in den Öffnungen oder Aufnahmen der Haltestruktur ohne größeren Kraftaufwand verstellt werden, insbesondere axial vorgeschoben oder gedreht werden.

Selbstverständlich kann die Haltestruktur (der Träger) im Sinne der vorliegenden Erfindung auch aus einem thermoplastischen, duroplastischen oder elastomeren Kunststoff ausgebildet sein, wobei zumindest Abschnitte der Haltestruktur bzw. des Trägers mit einer reibreduzierenden Beschichtung versehen sind, um das Einführen und die Entnahme der Behälter zu erleichtern.

## Patentansprüche

1. Verfahren zum Verschließen von Behältern (2), die eine Substanz für medizinische, pharmazeutische oder kosmetische Anwendungen enthalten, wobei die Behälter mittels einer Fördereinrichtung zur Behandlung oder Verarbeitung automatisiert an zumindest einer Prozessstation vorbeigeführt werden oder diese durchlaufen, bei welchem Verfahren
eine Mehrzahl von Behältern (2) von der Fördereinrichtung gefördert werden, während diese an einem Träger (25; 134) gemeinsam in einer regelmäßigen Anordnung gehalten werden, und
die an dem Träger (25; 134) gehaltenen Behälter (2) zur Behandlung oder Verarbeitung an oder in der zumindest einen Prozessstation in eine angehobene Position angehoben werden und nach der Behandlung oder Verarbeitung abgesenkt werden, um an dem Träger erneut in der regelmäßigen Anordnung gehalten zu werden, wobei
die Behandlung oder Verarbeitung das Befüllen der Behälter (2) mit der Substanz umfasst, während diese an dem Träger gehalten sind, und den Schritt, wonach die Behälter (2) vor dem Befüllen und nach dem Befüllen gewogen werden,
wobei das Verfahren ferner den Schritt des Verschließens der Behälter (2) umfasst, wobei der Schritt des Verschließens der Behälter die folgenden Schritte umfasst:
Aufdrücken von Kappen (198) auf die oberen Ränder der Behälter (2) vor oder nach dem Befüllen, und
Gegeneinanderdrücken von Ablageflächen, auf denen die Böden der Behälter (2) unmittelbar aufliegen, sodass die Kappen (198) auf die Behälter herabgedrückt und diese somit steril verschlossen werden;
wobei die Behälter (2) in Öffnungen oder Aufnahmen des Trägers in der regelmäßigen Anordnung gehalten werden, wobei den Öffnungen oder Aufnahmen jeweils zumindest zwei elastische Haltezungen (140) zugeordnet sind und wobei die Behälter (2) vor dem Herabdrücken der Kappen (198) jeweils von den Haltezungen (140) im Bereich eines verengten Halsabschnitts unterhalb eines verbreiterten oberen Rands (6) am oberen Endes des jeweiligen Behälters mit radialem Spiel und mit axialem Spiel oder mit radialem Spiel und axial frei verschiebbar gehalten werden.

2. Verfahren nach Anspruch 1, wobei die Innenvolumina der Behälter (2) über die bereits auf die oberen Ränder der Behälter (2) aufgesetzten Kappen mit der Umgebung kommunizieren, die Behälter über die bereits auf die oberen Ränder der Behälter (2) aufgesetzte Kappen mit der Substanz befüllt werden und die Behälter (2) nach Herunterdrücken der Kappen (198) verschlossen sind.

3. Verfahren nach Anspruch 1, wobei die Kappen (198) nach dem Befüllen der Behälter (2) auf die oberen Ränder der Behälter derart aufgedrückt werden, dass die Innenvolumina der Behälter (2) über die bereits auf die oberen Ränder der Behälter (2) aufgesetzte Kappen mit der Umgebung kommunizieren.

4. Verfahren nach einem der vorhergehenden Ansprüche, wobei die Behälter (2) so an dem Träger gehalten werden, dass Böden der Behälter von der Unterseite des Trägers her ungehindert und vollflächig zugänglich sind, wobei die Behälter (2) nach dem Befüllen einer Gefriertrocknung unterzogen werden, während diese an dem Träger gehalten werden, indem die Böden der Behälter (2) mit einer Kühlfläche oder einem Kühlfinger eines Gefriertrockners in einen unmittelbaren Kontakt gebracht werden, wobei die Innenvolumen der Behälter (2) während der Gefriertrocknung über die bereits auf die oberen Ränder der Behälter (2) aufgesetzte Kappen mit der Umgebung kommunizieren.

5. Verfahren nach Anspruch 4, wobei mehrere Kühlflächen des Gefriertrockners oder Ablageflächen mit den darauf unmittelbar aufliegenden Behältern gestapelt übereinander angeordnet sind und die Kühlflächen des Gefriertrockners oder Ablageflächen gegeneinander gedrückt werden, um die Kappen auf die Behälter (2) herabzudrücken und die Behälter mit den Kappen (198) zu verschließen.

6. Verfahren nach einem der vorhergehenden Ansprüche, wobei am oberen Ende der Haltezungen (140) Einführschrägen (140d) ausgebildet sind, welche jeweils in eine von den Haltezungen radial einwärts vorstehende Haltenase (140e) zum Halten der Behälter übergehen, wobei die Behälter (2) vor dem Herabdrücken der Kappen (198) jeweils im Bereich des verengten Halsabschnitts unterhalb des verbreiterten oberen Rands (6) am unteren Rand der vorstehenden Haltenasen (140e) mit radialem Spiel abgestützt sind.

7. Verfahren nach Anspruch 6, wobei die Kappen (198) nach dem Herabdrücken jeweils den verbreiterten oberen Rand (6) der Behälter (2) umgreifen und jeweils am unteren Rand der vorstehenden Haltenasen (140e) abgestützt sind.

## Claims

1. A process for sealing containers (2) that contain substances for medical, pharmaceutical or cosmetic applications, wherein the containers are conveyed automatically, by means of a conveyor, past at least one processing station or pass it for the treatment or processing, in which process
a plurality of containers (2) is conveyed by the conveyor while being held by a carrier (25; 134) in a regular arrangement, and
the containers held by the carrier (25; 134) are raised to a raised position for the treatment or processing at or in the respective processing station and are lowered after the treatment or processing, to be held again by the carrier in the regular arrangement, wherein
the treatment or processing comprises the filling of the containers (2) with the substance while being held by the carrier, and the step of weighing the containers (2) before the filling and after the filling,
wherein the process further comprises the step of sealing the containers (2), wherein the step of sealing the containers comprises the following steps:
pushing caps (198) onto the upper rims of the containers (2) before or after the filling, and
pressing supporting surfaces on which the bottoms of the containers (2) are directly supported against each other so that the caps (198) are pushed down onto the containers and these are thereby sterile sealed;
wherein the containers (2) are supported in openings or apertures of the carrier in the regular arrangement, wherein at least two resilient holding tabs (140) are respectively associated with the openings or apertures and wherein the containers (2) are respectively supported by the holding tabs (140) in the region of a constricted neck portion below an expanded upper rim (6) at an upper end of the respective container with a radial clearance and with an axial clearance or with a radial clearance and freely displaceable in axial direction before the pushing down of the caps (198)

2. The process according to claim 1, wherein the internal volumes of the containers (2) communicate with the environment via the caps positioned on the upper rims of the containers (2), the containers are filled with the substance via the caps positioned on the upper rims of the containers (2) and the containers (2) are sealed after pushing down the caps (198).

3. The process according to claim 1, wherein the caps (198) are pushed onto the upper rims of the containers after the filling of the containers (2) such that the internal volumes of the containers (2) communicate with the environment via the caps positioned on the upper rims of the containers (2).

4. The process according to any of the preceding claims, wherein the containers (2) are supported by the carrier such that the bottoms of the containers are freely and completely accessible from the underside of the carrier, wherein the containers (2) are subjected to a freeze-drying process after the step of filling while being held by the carrier by bringing the bottoms of the containers (2) into direct contact with a cooling surface or a cooling finger of a freeze-dryer, wherein the internal volumes of the containers (2) communicate with the environment via the caps positioned on the upper rims of the containers (2) during the freeze-drying.

5. The process according to claim 4, wherein a plurality of cooling surfaces or cooling fingers of the freeze-dryer together with the containers directly supported thereon are stacked one above the other and wherein the cooling surfaces or cooling fingers of the freeze-dryer are pressed against each other for pushing down the caps of the containers (2) and sealing the containers with the caps (198).

6. The process according to any of the preceding claims, wherein slanted insertion surfaces (140d) are formed at upper ends of the holding tabs (140), which are respectively followed by holding noses (140e) projecting from the holding tabs radially inwards, for supporting the containers, wherein the containers (2) are respectively supported with radial clearance in the region of the constricted neck portion below the expanded upper rim (6) at a bottom rim of the protruding holding noses (140e) before pushing down the caps (198).

7. The process according to claim 6, wherein the caps (198) each embrace the expanded upper rims (6) of the containers (2) and each are supported by the bottom rim of the protruding holding noses (140e) after being pushed down.

## Revendications

1. Procédé de scellement de récipients (2) contenant des substances pour des applications cosmétiques, médicales ou pharmaceutiques, dans lequel les récipients sont transportés automatiquement, au moyen d'un convoyeur, au delà d'au moins une station de traitement ou en passant par celle-ci pour une préparation ou traitement, dans lequel
plusieurs récipients (2) sont transportés par le convoyeur tout en étant maintenus sur un support (25; 134) au sein d'une disposition ordonnée, et
les récipients maintenus par le support (25 ; 134) sont levés vers une position haute pour un traitement par ou dans la station de traitement respective et sont abaissés à la suite du traitement, afin d'être à nouveau maintenus par le support au sein de la disposition ordonnée, dans lequel
le traitement comporte le remplissage des récipients (2) avec une substance tout en étant maintenu par le support, et l'étape de pesée des récipients (2) avant et après remplissage,
dans lequel le traitement comporte en outre l'étape de scellement des récipients (2), dans laquelle l'étape de scellement des récipients comporte les étapes suivantes :
pousser des bouchons (198) sur les bords supérieurs des récipients (2) avant ou après remplissage, et
presser les surfaces de support sur lesquelles les fonds des récipients (2) sont directement soutenus les uns contre les autres de façon à pousser les bouchons vers des récipients pour un scellement résultant stérile ;
dans lequel les récipients (2) sont soutenus dans des trous ou ouvertures du support au sein de la disposition ordonnée, dans lesquels au moins deux languettes de maintien (140) sont respectivement associées avec les trous ou ouvertures et dans lequel les récipients (2) sont respectivement soutenus par les languettes de maintien (140) dans la région d'une partie de col rétréci en dessous d'un bord supérieur élargi (6) au niveau d'une extrémité supérieure du récipient respectif avec une libération radiale et avec une libération axiale ou avec un jeu radial et un jeu axial ou avec un jeu radial et librement mobile suivant une direction axiale avant la poussée vers le bas des bouchons (198).

2. Le procédé selon la revendication 1, dans lequel les volumes internes des récipients (2) communiquent avec l'environnement via les bouchons positionnés sur les bords supérieurs des récipients (2), les récipients sont remplis avec la substance via les bouchons positions sur les bords supérieurs des récipients (2) et les récipients (2) sont scellés à la suite de la poussée vers le bas des bouchons (198).

3. Le procédé selon la revendication 1, dans lequel les bouchons (198) sont poussés sur les bords supérieurs des récipients à la suite du remplissage des récipients (2) de telle manière que les volumes internes des récipients (2) communiquant avec l'environnement via les bouchons positionnés sur les bords supérieurs des récipients (2).

4. Le procédé selon l'une quelconque des revendications précédentes, dans lequel les récipients (2) sont maintenus sur le support de telle manière que les fonds des récipients sont librement et complètement accessibles depuis la face inférieure du support, dans lequel les récipients (2) font l'objet d'un procédé de lyophilisation à la suite du remplissage tout en étant maintenus par le support en amenant les fonds des récipients (2) en contact direct avec une surface de refroidissement ou un doigt de refroidissement d'un appareil de lyophilisation, dans lequel les volumes intérieurs des récipients (2) communiquent avec l'environnement via les bouchons positionnés sur les bords supérieurs des récipients durant la lyophilisation.

5. Le procédé selon la revendication 4, dans lequel une pluralité de surfaces de refroidissement ou de doigts de refroidissement de l'appareil de lyophilisation, avec les récipients qui sont directement soutenus sur ceux-ci, sont empilés les uns sur les autres et dans lequel les surfaces de refroidissement ou les doigts de refroidissement de l'appareil de lyophilisation sont pressés les uns sur les autres pour pousser vers le bas les bouchons des récipients (2) et sceller les récipients au moyen des bouchons (198).

6. Le procédé selon l'une quelconque des revendications précédentes, dans lequel des surfaces d'insertion chanfreinées (140d) sont formées à des extrémités supérieures des languettes de maintien (140), qui sont respectivement suivies de pattes de maintien (140 e) se projetant radialement depuis l'intérieur les languettes de maintien (140), pour soutenir les récipients (2), dans lequel les récipients (2) sont respectivement soutenus avec un jeu radial dans la région de la partie de col rétréci en dessous du bord supérieur élargi (6) au niveau d'un bord inférieur des pattes de maintien en saillie (140 e) préalablement à la poussée vers le bas des bouchons (198).

7. Le procédé selon la revendication 6, dans lequel les bouchons (198) entourent chacun les bords supérieurs élargis (6) des récipients (2) et sont chacun soutenus par le bord inférieur des pattes de maintien en saillie (140 e) postérieurement à leur poussée vers le bas.
